(19) Europäisches Patentamt
European Patent Office
Office européen des brevets

(11) **EP 4 792 817 A1**

(12) **EUROPEAN PATENT APPLICATION**
published in accordance with Art. 153(4) EPC

(43) Date of publication:
**19.08.2026 Bulletin 2026/34**

(21) Application number: **24876688.3**

(22) Date of filing: **12.10.2024**

(51) International Patent Classification (IPC):
***A61K 31/7048*** *(2006.01)* ***A61P 35/00*** *(2006.01)*

(52) Cooperative Patent Classification (CPC):
**A61K 31/7048; A61P 35/00**

(86) International application number:
**PCT/CN2024/124477**

(87) International publication number:
**WO 2025/077883 (17.04.2025 Gazette 2025/16)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **13.10.2023 CN 202311331579**

(71) Applicant: **EnKang Pharmaceuticals (Guangzhou), Ltd.**
**Guangzhou, Guangdong 511400 (CN)**

(72) Inventors:
- **LIANG, Chun**
  **Guangzhou, Guangdong 511400 (CN)**
- **CHEUNG, Man Hei**
  **Guangzhou, Guangdong 511400 (CN)**

(74) Representative: **Murgitroyd & Company**
**165-169 Scotland Street**
**Glasgow G5 8PL (GB)**

(54) **COMPOUND AND METHOD FOR TREATING AND/OR PREVENTING SKIN CANCER OR PRECANCEROUS LESION THEREOF**

(57) The present application relates to 17β-neriifolin or a pharmaceutically acceptable salt, racemate, stereoisomer, enantiomer, diastereomer, tautomer, metabolite, solvate or prodrug thereof, and a method thereof for treating and/or preventing skin cancer or precancerous lesions thereof. The compound can penetrate through the skin and act directly on skin lesion sites, and has an improved efficacy and a relatively high safety.

SCL-1 human skin cancer
Model control group
Drug substance group
Imiquimod cream group
Gel group I
Gel group II
Gel group III
Gel group IV
Volume（mm³）
3500
3000
2500
2000
1500
1000
500
0
0D
3D
6D
9D
12D
15D
D18
D21
Days

FIG. 2



Processed by Luminess, 75001 PARIS (FR)

## Description

The present application claims priority and benefits to the patent application No.202311331579.5 filed with the China National Intellectual Property Administration on October 13, 2023, which is incorporated herein by reference in its entirety.

## TECHNICAL FIELD

The present application relates to the technical field of pharmaceuticals, and particularly to a compound and method for treating and/or preventing skin cancer or precancerous lesions thereof.

## BACKGROUND

It has been reported that neriifolin can target beclin1, inhibit the formation of LC3-associated phagosomes, and ameliorate the progression of experimental autoimmune encephalomyelitis (EAE). Patent No. CN 104736157 A documents that 17β-neriifolin can induce cell cycle arrest and apoptosis in human liver cancer HepG2 cells.

Skin cancer is the most prevalent malignant tumor in humans worldwide, and is estimated to constitute about 32% of annually diagnosed cancer cases. Skin cancer is classified into melanoma skin cancer and non-melanoma skin cancer, with the latter being the most common form and including basal cell carcinoma, cutaneous squamous cell carcinoma, etc.

Surgical excision and radiotherapy are traditional therapeutic approaches for localized non-melanoma skin cancer. However, the recurrence rate is about 30% when either surgery or radiotherapy is used alone. Furthermore, some patients are not suitable candidates for surgery or radiotherapy because of disfigurement, inability to achieve radical excision due to anatomical limitations, or other various forms of cutaneous adverse reactions. Most of the anticancer drugs currently used in clinical practice are administered orally or via injection, making them unsuitable for making topical formulations that act directly on skin lesions. Consequently, they cannot be utilized for the treatment of skin cancer. Known topical drugs for skin cancer treatment, such as 5-Fluorouracil and Imiquimod, have limited therapeutic efficacy. There remains an unmet need for the development of novel drugs suitable for treating skin cancer or precancerous lesions thereof.

## SUMMARY OF THE INVENTION

In one aspect, the present application relates, at least in part, to a compound, which is 17β-neriifolin or a pharmaceutically acceptable salt, racemate, stereoisomer, enantiomer, diastereomer, tautomer, metabolite, solvate or prodrug thereof, for use in the treatment and/or prevention of skin cancer or precancerous lesions thereof via topical administration.

In one aspect, the present application relates, at least in part, to a method for treating and/or preventing skin cancer or precancerous lesions thereof, which comprises administering 17β-neriifolin or a pharmaceutically acceptable salt, racemate, stereoisomer, enantiomer, diastereomer, tautomer, metabolite, solvate or prodrug thereof.

In one aspect, the present application relates, at least in part, to the method for treating and/or preventing skin cancer or precancerous lesions thereof, which comprises administering to a subject in need thereof a therapeutically effective amount of 17β-neriifolin or a pharmaceutically acceptable salt, racemate, stereoisomer, enantiomer, diastereomer, tautomer, metabolite, solvate or prodrug thereof.

In one aspect, the skin cancer includes melanoma and/or non-melanoma skin cancer. In one aspect, the non-melanoma skin cancer is basal cell carcinoma and/or cutaneous squamous cell carcinoma.

In one aspect, 17β-neriifolin or a pharmaceutically acceptable salt, racemate, stereoisomer, enantiomer, diastereomer, tautomer, metabolite, solvate or prodrug thereof is administered to the affected area(s) of the subject(s) at a dose of: about 0.0003 mg to about 100 mg; about 0.008 mg to about 90 mg; about 0.1 mg to about 80 mg; about 0.1 mg to about 70 mg; about 0.1 mg to about 60 mg; about 0.1 mg to about 50 mg; about 0.1 mg to about 40 mg; about 0.1 mg to about 30 mg; about 0.1 mg to about 20 mg; about 0.1 mg to about 15 mg; about 0.1 mg to about 10 mg; about 0.1 mg to about 8 mg; about 0.2 mg to about 5 mg; or about 0.5 mg to about 2.5 mg.

In one aspect, 17β-neriifolin or a pharmaceutically acceptable salt, racemate, stereoisomer, enantiomer, diastereomer, tautomer, metabolite, solvate or prodrug thereof is administered to the affected area(s) of the subject(s) at a dose of: about 0.02 mg, about 0.05 mg, about 0.1 mg, about 0.2 mg, about 0.3 mg, about 0.4 mg, about 0.5 mg, about 0.6 mg, about 0.7 mg, about 0.8 mg, about 0.9 mg, about 1 mg, about 1.1 mg, about 1.2 mg, about 1.3 mg, about 1.4 mg, about 1.5 mg, about 1.6 mg, about 1.7 mg, about 1.8 mg, about 1.9 mg, about 2.0 mg, about 2.1 mg, about 2.2 mg, about 2.3 mg, about 2.4 mg, about 2.5 mg, about 2.6 mg, about 2.7 mg, about 2.8 mg, about 2.9 mg, about 3 mg, about 4 mg, or about 5 mg.

In one aspect, 17β-neriifolin or a pharmaceutically acceptable salt, racemate, stereoisomer, enantiomer, diastereomer, tautomer, metabolite, solvate or prodrug thereof is administered to the affected area(s) of the subject(s) at a dose of: about 0.0003 $mg/cm^2$ to about 10 $mg/cm^2$; preferably about 0.001 $mg/cm^2$ to about 1 $mg/cm^2$; preferably about 0.002 $mg/cm^2$ to about 0.5 $mg/cm^2$; preferably about 0.004 $mg/cm^2$ to about 0.25 $mg/cm^2$; preferably about 0.008 $mg/cm^2$ to about 0.2 $mg/cm^2$; preferably about 0.01 $mg/cm^2$ to about 0.18 $mg/cm^2$; preferably about 0.016 $mg/cm^2$ to about 0.16 $mg/cm^2$; preferably about 0.02 $mg/cm^2$ to about 0.15 $mg/cm^2$; preferably about 0.025 $mg/cm^2$ to about 0.14 $mg/cm^2$; preferably about 0.027 $mg/cm^2$ to about 0.13 $mg/cm^2$; preferably about 0.03 $mg/cm^2$ to about 0.125 $mg/cm^2$; preferably about 0.035 $mg/cm^2$ to about 0.12 $mg/cm^2$; preferably about 0.04 $mg/cm^2$ to about 0.1 $mg/cm^2$.

In one aspect, 17β-neriifolin or a pharmaceutically acceptable salt, racemate, stereoisomer, enantiomer, diastereomer, tautomer, metabolite, solvate or prodrug thereof is administered to the affected area(s) of the subject(s) at a dose of: about 0.001 $mg/cm^2$, about 0.002 $mg/cm^2$, about 0.003 $mg/cm^2$, about 0.004 $mg/cm^2$, about 0.005 $mg/cm^2$, about 0.006 $mg/cm^2$, about 0.007 $mg/cm^2$, about 0.008 $mg/cm^2$, about 0.009 $mg/cm^2$, about 0.01 $mg/cm^2$, about 0.015 $mg/cm^2$, about 0.02 $mg/cm^2$, about 0.025 $mg/cm^2$, about 0.03 $mg/cm^2$, about 0.035 $mg/cm^2$, about 0.04 $mg/cm^2$, about 0.045 $mg/cm^2$, about 0.05 $mg/cm^2$, about 0.055 $mg/cm^2$, about 0.06 $mg/cm^2$, about 0.065 $mg/cm^2$, about 0.07 $mg/cm^2$, about 0.075 $mg/cm^2$, about 0.08 $mg/cm^2$, about 0.085 $mg/cm^2$, about 0.09 $mg/cm^2$, about 0.095 $mg/cm^2$, about 0.1 $mg/cm^2$, about 0.15 $mg/cm^2$, about 0.2 $mg/cm^2$, about 0.25 $mg/cm^2$, about 0.3 $mg/cm^2$, about 0.35 $mg/cm^2$, about 0.4 $mg/cm^2$, about 0.45 $mg/cm^2$, about 0.5 $mg/cm^2$, about 0.55 $mg/cm^2$, about 0.6 $mg/cm^2$, or about 0.65 $mg/cm^2$.

In one aspect, the affected area(s) of the subject(s) is/are about 0.01 $cm^2$ to about 300 $cm^2$; preferably, the affected area(s) of the subject(s) is/are: about 1 $cm^2$ to about 200 $cm^2$, about 1 $cm^2$ to about 100 $cm^2$, about 1 $cm^2$ to about 75 $cm^2$, about 1 $cm^2$ to about 50 $cm^2$, or about 1 $cm^2$ to about 25 $cm^2$; about 10 $cm^2$ to about 200 $cm^2$, about 10 $cm^2$ to about 100 $cm^2$, about 10 $cm^2$ to about 75 $cm^2$, about 10 $cm^2$ to about 50 $cm^2$, or about 10 $cm^2$ to about 25 $cm^2$; about 25 $cm^2$ to about 200 $cm^2$, about 25 $cm^2$ to about 100 $cm^2$, or about 25 $cm^2$ to about 75 $cm^2$; about 25 $cm^2$ to about 90 $cm^2$, about 25 $cm^2$ to about 80 $cm^2$, or about 25 $cm^2$ to about 70 $cm^2$, about 25 $cm^2$ to about 60 $cm^2$, about 25 $cm^2$ to about 50 $cm^2$, about 25 $cm^2$ to about 40 $cm^2$, or about 25 $cm^2$ to about 30 $cm^2$. Preferably, the affected area(s) of the subject(s) is/are about 0.2 $cm^2$, about 0.5 $cm^2$, about 1 $cm^2$, about 2 $cm^2$, about 3 $cm^2$, about 4 $cm^2$, about 5 $cm^2$, about 6 $cm^2$, about 7 $cm^2$, about 8 $cm^2$, about 9 $cm^2$, about 10 $cm^2$, about 11 $cm^2$, about 12 $cm^2$, about 13 $cm^2$, about 14 $cm^2$, about 15 $cm^2$, about 16 $cm^2$, about 17 $cm^2$, about 18 $cm^2$, about 19 $cm^2$, about 20 $cm^2$, about 25 $cm^2$, about 30 $cm^2$, about 35 $cm^2$, about 40 $cm^2$, about 45 $cm^2$, about 50 $cm^2$, about 55 $cm^2$, about 60 $cm^2$, about 65 $cm^2$, about 70 $cm^2$, about 75 $cm^2$, about 80 $cm^2$, about 85 $cm^2$, about 90 $cm^2$, about 95 $cm^2$, or about 100 $cm^2$.

In one aspect, the affected area(s) of the subject(s) is/are on the skin.

In one aspect, the affected area(s) of the subject(s) is/are located at one or more positions independently selected from the scalp, forehead, forearm, face, nose, ears, eyelids, lips, neck, arms, hands, trunk, legs, and feet.

In one aspect, the subject has more than one affected area. In one aspect, 17β-neriifolin or a pharmaceutically acceptable salt, racemate, stereoisomer, enantiomer, diastereomer, tautomer, metabolite, solvate or prodrug thereof is administered to the patient via topical administration.

In one aspect, 17β-neriifolin or a pharmaceutically acceptable salt, racemate, stereoisomer, enantiomer, diastereomer, tautomer, metabolite, solvate or prodrug thereof is formulated into a pharmaceutical composition suitable for topical use.

In one aspect, the content of 17β-neriifolin or a pharmaceutically acceptable salt, racemate, stereoisomer, enantiomer, diastereomer, tautomer, metabolite, solvate or prodrug thereof is about 0.025 wt% to 4 wt%; preferably about 0.05 wt% to 3 wt%; preferably about 0.2 wt% to 0.8 wt%; preferably about 0.4 wt% to 0.8 wt%; preferably about 0.075 wt%, about 0.1 wt%, about 0.125 wt%, about 0.15 wt%, about 0.175 wt%, about 0.2 wt%, about 0.225 wt%, about 0.25 wt%, about 0.275 wt%, about 0.3 wt%, about 0.325 wt%, about 0.35 wt%, about 0.375 wt%, about 0.4 wt%, about 0.425 wt%, about 0.45 wt%, about 0.475 wt%, about 0.5 wt%, about 0.525 wt%, about 0.55 wt%, about 0.575 wt%, about 0.6 wt%, about 0.625 wt%, about 0.65 wt%, about 0.675 wt%, about 0.7 wt%, about 0.725 wt%, about 0.75 wt%, about 0.775 wt%, about 0.8 wt%, about 0.825 wt%, about 0.85 wt%, about 0.875 wt%, about 0.9 wt%, about 0.925 wt%, about 0.95 wt%, about 0.975 wt%, about 1 wt%, about 1.25 wt%, about 1.5 wt%, about 1.75 wt%, about 2.0 wt%, about 2.25 wt%, about 2.5 wt%, or about 2.75 wt%, based on the total weight of the pharmaceutical composition.

In one aspect, the pharmaceutical composition is a gel.

In one aspect, the pharmaceutically acceptable excipients in the gel optionally include a gel matrix. The gel matrix is selected from at least one of sodium carboxymethyl cellulose, carbomer, gelatin, and alginate. In one aspect, the content of the gel matrix is about 0.5 wt% to 5 wt%; preferably about 1 wt% to 4 wt%; preferably about 1 wt% to 3 wt%; more preferably about 1.5 wt%, about 2 wt%, about 2.5 wt%, or about 3 wt%, based on the total weight of the pharmaceutical composition.

In one aspect, the pharmaceutically acceptable excipients in the gel optionally include a humectant. The humectant is selected from at least one of alcoholic humectants; preferably, the humectant is glycerol. In one aspect, the content of the humectant is about 5 wt% to 35 wt%; preferably about 10 wt% to 30 wt%; preferably about 15 wt% to 25 wt%; more preferably about 15 wt%, about 20 wt%, about 25 wt%, or about 30 wt%, based on the total weight of the

pharmaceutical composition.

In one aspect, the pharmaceutically acceptable excipients in the gel optionally include a solvent; preferably, the solvent is selected from at least one of ethanol, propylene glycol, and benzyl alcohol; the ethanol is 95% ethanol or anhydrous ethanol. In one aspect, the content of the solvent is about 20 wt% to 75 wt%; preferably about 25 wt% to 70 wt%; more preferably about 30 wt%, about 35 wt%, about 40 wt%, about 45 wt%, about 50 wt%, about 55 wt%, about 60 wt%, or about 65 wt%, based on the total weight of the pharmaceutical composition.

In one aspect, the pharmaceutically acceptable excipients in the gel optionally include water, preferably purified water, distilled water, or deionized water. In one aspect, the content of the water is about 20 wt% to 75 wt%; preferably about 25 wt% to 70 wt%; more preferably about 30 wt%, about 35 wt%, about 40 wt%, about 45 wt%, about 50 wt%, about 55 wt%, about 60 wt%, or about 65 wt%, based on the total weight of the pharmaceutical composition.

In one aspect, the pharmaceutically acceptable excipients in the gel optionally include a penetration enhancer. In one aspect, the penetration enhancer is selected from at least one of azone, borneol, Tween 80, sodium dodecyl sulfate, and poloxamer; more preferably, the penetration enhancer is azone or borneol. In one aspect, the content of the penetration enhancer is about 0 wt% to 5 wt%; preferably about 0.1 wt% to 4 wt%; more preferably about 0.25 wt%, about 0.5 wt%, about 0.75 wt%, about 1 wt%, about 1.25 wt%, about 1.5 wt%, about 1.75 wt%, about 2 wt%, about 2.25 wt%, about 2.5 wt%, about 2.75 wt%, about 3 wt%, about 3.25 wt%, about 3.5 wt%, or about 3.75 wt%, based on the total weight of the pharmaceutical composition.

In one aspect, the pharmaceutically acceptable excipients optionally include a preservative. The preservative is a paraben preservative and/or butylated hydroxytoluene; preferably, the paraben preservative is methylparaben and/or ethylparaben. In one aspect, the content of the preservative is about 0 wt% to 4 wt%; preferably about 0.1 wt% to 3 wt%; more preferably about 0.25 wt%, about 0.5 wt%, about 0.75 wt%, about 1 wt%, about 1.25 wt%, about 1.5 wt%, about 1.75 wt%, about 2 wt%, about 2.25 wt%, about 2.5 wt%, or about 2.75 wt%, based on the total weight of the pharmaceutical composition.

In one aspect, the pharmaceutical composition comprises:

about 0.2 wt% to 0.8 wt% 17β-neriifolin or a pharmaceutically acceptable salt, racemate, stereoisomer, enantiomer, diastereomer, tautomer, metabolite, solvate or
prodrug thereof; and/or
about 1.5 wt% gel matrix; and/or
about 40 wt% solvent; and/or
about 0.5 wt% penetration enhancer; and/or
about 20 wt% humectant;
the balance being water.

In one aspect, the pharmaceutical composition is a topical composition comprising 17β-neriifolin free base, wherein the content of 17β-neriifolin free base, as determined by HPLC, is greater than about 90%, preferably greater than about 90.5%, greater than about 91%, greater than about 91.5%, greater than about 92%, greater than about 92.5%, greater than about 93%, greater than about 93.5%, greater than about 94%, greater than about 94.5%, greater than about 95%, greater than about 95.5%, greater than about 96%, greater than about 96.5%, greater than about 97%, greater than about 97.5%, greater than about 98%, greater than about 98.5%, greater than about 99%, greater than about 99.5%, greater than about 99.6%, greater than about 99.7%, greater than about 99.8%, or greater than about 99.9%.

In one aspect, the dosage form of the topical composition includes a powder, a spray, an ointment, an emulgel, a paste, a cream, a lotion, a gel, a solution, a patch, and an inhalant.

In one aspect, the dosage form of the topical composition includes a gel.

In one aspect, the amount of the pharmaceutical composition administered to an affected skin area(s) ranges/range from about 0.001 g/cm$^2$ of skin surface area to about 0.5 g/cm$^2$ of skin surface area; preferably about 0.0015 g/cm$^2$ of skin surface area to about 0.2 g/cm$^2$ of skin surface area; preferably about 0.0015 g/cm$^2$ of skin surface area to about 0.15 g/cm$^2$ of skin surface area; preferably about 0.002 g/cm$^2$ of skin surface area to about 0.125 g/cm$^2$ of skin surface area; preferably about 0.003 g/cm$^2$ of skin surface area to about 0.1 g/cm$^2$ of skin surface area; preferably about 0.004 g/cm$^2$ of skin surface area; about 0.005 g/cm$^2$ of skin surface area; about 0.006 g/cm$^2$ of skin surface area; about 0.007 g/cm$^2$ of skin surface area; about 0.008 g/cm$^2$ of skin surface area; about 0.009 g/cm$^2$ of skin surface area; about 0.01 g/cm$^2$ of skin surface area; about 0.0125 g/cm$^2$ of skin surface area; about 0.015 g/cm$^2$ of skin surface area; about 0.0175 g/cm$^2$ of skin surface area; about 0.02 g/cm$^2$ of skin surface area; about 0.03 g/cm$^2$ of skin surface area; about 0.04 g/cm$^2$ of skin surface area; about 0.05 g/cm$^2$ of skin surface area; about 0.06 g/cm$^2$ of skin surface area; about 0.07 g/cm$^2$ of skin surface area; about 0.08 g/cm$^2$ of skin surface area; or about 0.09 g/cm$^2$ of skin surface area.

In one aspect, 17β-neriifolin or a pharmaceutically acceptable salt, racemate, stereoisomer, enantiomer, diastereomer, tautomer, metabolite, solvate or prodrug thereof is administered daily, at one-day intervals, at two-day intervals, at three-day intervals, at four-day intervals, at five-day intervals, at six-day intervals, or at seven-day intervals.

Generally, on the day of administration, one, two, three, or four administrations are performed.

In one aspect, 17β-neriifolin or a pharmaceutically acceptable salt, racemate, stereoisomer, enantiomer, diastereomer, tautomer, metabolite, solvate or prodrug thereof is administered once a week, once every six days, once every five days, once every four days, once every three days, once every two days, once a day, twice a day, three times a day, or four times a day; preferably once every two days or once a day.

In one aspect, the administration cycle of 17β-neriifolin or a pharmaceutically acceptable salt, racemate, stereoisomer, enantiomer, diastereomer, tautomer, metabolite, solvate or prodrug thereof may be 1 to 100 days, preferably 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, or 60 days.

In one aspect, 17β-neriifolin or a pharmaceutically acceptable salt, racemate, stereoisomer, enantiomer, diastereomer, tautomer, metabolite, solvate or prodrug thereof may be administered at a frequency of once or twice daily continuously for more than one day per week, followed by discontinuation of the administration for the rest of the week. In one aspect, the administration is performed once or twice daily every other day. In one aspect, the administration is performed once or twice daily every three, four, five, six, or seven days. In one aspect, the administration is performed once or twice daily for two days in a row every three, four, five, six, or seven days. In one aspect, the administration is performed once or twice daily for three days in a row every four, five, six, or seven days. In one aspect, the administration is performed once or twice daily for four days in a row every five, six, or seven days. In one aspect, the administration is performed until skin cancer or precancerous lesions thereof are completely cleared. In one aspect, the administration is topical administration.

In one aspect, the drug is kept in contact with the skin for about 0.5 h to about 24 hrs, optionally about 1 h, about 1.5 hrs, about 2 hrs, about 2.5 hrs, about 3 hrs, about 3.5 hrs, about 4 hrs, about 4.5 hrs, about 5 hrs, about 5.5 hrs, about 6 hrs, about 6.5 hrs, about 7 hrs, about 7.5 hrs, about 8 hrs, about 8.5 hrs, about 9 hrs, about 9.5 hrs, about 10 hrs, about 11 hrs, about 12 hrs, about 13 hrs, about 14 hrs, about 15 hrs, about 16 hrs, about 17 hrs, about 18 hrs, about 19 hrs, about 20 hrs, about 21 hrs, about 22 hrs, about 23 hrs, or about 24 hrs per administration.

In one aspect, compared to other treatments of skin cancer or precancerous lesions thereof, the administration of 17β-neriifolin or a pharmaceutically acceptable salt, racemate, stereoisomer, enantiomer, diastereomer, tautomer, metabolite, solvate or prodrug thereof reduces the number and/or severity of local skin reactions or other adverse effects in the subject(s).

In one aspect, compared to other treatments of skin cancer or precancerous lesions thereof, the administration of 17β-neriifolin or a pharmaceutically acceptable salt, racemate, stereoisomer, enantiomer, diastereomer, tautomer, metabolite, solvate or prodrug thereof reduces the number of subjects experiencing local skin reactions or other adverse effects.

In one aspect, the local skin reaction is selected from vesicle formation, pustule formation, erosion, ulceration, redness, swelling, scaling, desquamation, induration, dryness, pus, blistering, and the like.

In one aspect, the other side effect is selected from pain at the site of administration, pruritus at the site of administration, irritation at the site of administration, swelling at the site of administration, burning sensation at the site of administration, infection at the site of administration, edema around the eyes, nasopharyngitis, chills, sore throat, blepharoptosis, eye puffiness, hypopigmentation, hyperpigmentation, headache, and the like.

In one aspect, the present application relates, at least in part, to 17β-neriifolin or a pharmaceutically acceptable salt, racemate, stereoisomer, enantiomer, diastereomer, tautomer, metabolite, solvate or prodrug thereof for use (e.g., topical use) in the treatment and/or prevention of skin cancer or precancerous lesions thereof. In some aspects, 17β-neriifolin or a pharmaceutically acceptable salt, racemate, stereoisomer, enantiomer, diastereomer, tautomer, metabolite, solvate or prodrug thereof is used at the dose, on the administration schedule, and/or in one or more affected areas of the subject(s) in need thereof, as described herein.

In one aspect, the present application relates, at least in part, to use (e.g., topical use) of 17β-neriifolin or a pharmaceutically acceptable salt, racemate, stereoisomer, enantiomer, diastereomer, tautomer, metabolite, solvate or prodrug thereof in the treatment and/or prevention of skin cancer or precancerous lesions thereof. In some aspects, 17β-neriifolin or a pharmaceutically acceptable salt, racemate, stereoisomer, enantiomer, diastereomer, tautomer, metabolite, solvate or prodrug thereof is used at the dose, on the administration schedule, and/or in one or more affected areas of the subject(s) in need thereof, as described herein.

In one aspect, the present application relates, at least in part, to use of 17β-neriifolin or a pharmaceutically acceptable salt, racemate, stereoisomer, enantiomer, diastereomer, tautomer, metabolite, solvate or prodrug thereof in the preparation of a medicament for treating and/or preventing skin cancer or precancerous lesions thereof. In some aspects, 17β-neriifolin or a pharmaceutically acceptable salt, racemate, stereoisomer, enantiomer, diastereomer, tautomer, metabolite, solvate or prodrug thereof is used at the dose, on the administration schedule, and/or in one or more affected areas of the subject(s) in need thereof, as described herein.

In one aspect, the present application relates, at least in part, to a therapeutic agent for skin cancer or precancerous lesions thereof, comprising 17β-neriifolin or a pharmaceutically acceptable salt, racemate, stereoisomer,

enantiomer, diastereomer, tautomer, metabolite, solvate or prodrug thereof. In some aspects, 17β-neriifolin or a pharmaceutically acceptable salt, racemate, stereoisomer, enantiomer, diastereomer, tautomer, metabolite, solvate or prodrug thereof is used at the dose, on the administration schedule, and/or in one or more affected areas of the subject(s) in need thereof, as described herein.

In this text, the term "skin cancer" refers to malignant skin tumors, including melanoma and non-melanoma skin cancers. These cancers can be primary or secondary. In this context, the term also refers to metastatic lesions that originate from primary skin cancer or another cancer that has metastasized to the skin.

Basal cell carcinoma and cutaneous squamous cell carcinoma are examples of non-melanoma skin cancer. As used herein, the term "basal cell carcinoma" refers to a tumor with low-grade malignancy that originates from the basal cells of the skin, including nodular, superficial spreading, micronodular, or mixed types, etc. The term "Cutaneous squamous cell carcinoma" as used herein, also known as epidermoid carcinoma, is a malignant tumor that originates from the squamous epithelial cells in the epidermis or its appendages, and it accounts for 20% to 50% of skin cancers and exhibits higher invasiveness compared to basal cell carcinoma, with a greater propensity for local and distant metastases.

As used herein, the term "precancerous skin lesions" refers to epithelial lesions or abnormal proliferation, being a cancer precursor or potentially becoming a cancer precursor. This includes, but is not limited to, Bowen's disease, arsenical keratosis, radiodermatitis, leukoplakia, junctional nevus, etc.

The term "trunk" as used herein refers to a part of a subject/patient excluding the arms, legs, and head.

The phrase "until skin cancer or precancerous lesions thereof are cleared" as used herein refers to a condition in which the lesion(s) on a subject having skin cancer or precancerous lesions thereof have substantially or completely disappeared from the treated area(s) of the subject. In one embodiment, "substantially" in this context refers to the disappearance of more than 50% of skin cancer or precancerous lesions thereof from the treated area(s) of the subject. In another embodiment, "substantially" refers to the disappearance of more than 60% of skin cancer or precancerous lesions thereof from the treated area(s) of the subject. In another embodiment, "substantially" refers to the disappearance of more than 70% of skin cancer or precancerous lesions thereof from the treated area(s) of the subject. In another embodiment, "substantially" refers to the disappearance of more than 80% of skin cancer or precancerous lesions thereof from the treated area(s) of the subject. In another embodiment, "substantially" refers to the disappearance of more than 90% of skin cancer or precancerous lesions thereof from the treated area(s) of the subject. In another embodiment, "substantially" refers to the disappearance of more than 95% of skin cancer or precancerous lesions thereof from the treated area(s) of the subject. In another embodiment, "substantially" refers to the disappearance of more than 99% of skin cancer or precancerous lesions thereof from the treated area(s) of the subject.

As used throughout the present disclosure, the singular forms "a," "an," and "the" include plural referents, unless otherwise clearly indicated in the context. Thus, for example, reference to "a method" includes multiple such methods, and reference to "a dose" includes reference to one or more doses and equivalents thereof known to those skilled in the art, and so forth.

The term "comprising" is intended to mean that the method includes the enumerated elements but does not exclude other elements. "Consisting essentially of...", when used to define a method, shall mean the exclusion of other elements that have any fundamental significance to the combination when used for the intended purpose. Thus, a method consisting essentially of the elements defined herein does not exclude substantial method steps. "Consisting of..." means excluding multiple substantial method steps. Embodiments defined by each of these transitional terms fall within the scope of the present application.

Unless otherwise clearly specified, the terms "approximately" and "about" are synonymous. In one embodiment, "approximately" and "about" refer to ±5%, ±4.5%, ±4%, ±3.5%, ±3%, ±2.5%, ±2%, ±1.75%, ±1.5%, ±1.25%, ±1%, ±0.9%, ±0.8%, ±0.7%, ±0.6%, ±0.5%±0.4%, ±0.3%, ±0.2%, ±0.1%, ±0.09%, ±0.08%, ±0.07%, ±0.06%, ±0.05%, ±0.04%, ±0.03%, ±0.02%, or ±0.01% of the stated amount, value, or duration. In another embodiment, "approximately" and "about" refer to ±2.5%, ±2%, ±1.75%, ±1.5%, ±1.25%, ±1%, ±0.9%, ±0.8%, ±0.7%, ±0.6%, or ±0.5% of the listed amount, value, or duration. In another embodiment, "approximately" and "about" refer to ±1% of the listed amount, value, or duration. In another embodiment, "approximately" and "about" refer to ±0.5% of the listed amount, value, or duration. In another embodiment, "approximately" and "about" refer to ±0.1% of the listed amount, value, or duration.

The term "subject" includes any living organism having or at risk of developing skin cancer or precancerous lesions thereof. In one embodiment, the term "subject" refers to mammals having or at risk of developing skin cancer or precancerous lesions thereof. In one embodiment, the term "subject" refers to humans having or at risk of developing skin cancer or precancerous lesions thereof. Unless otherwise clearly indicated, the terms "patient(s)" and "subject(s)" are synonymous.

As used herein, the term "therapeutically effective amount" refers to an amount of an agent (e.g., 17β-neriifolin or a pharmaceutically acceptable salt, racemate, stereoisomer, enantiomer, diastereomer, tautomer, metabolite, solvate or prodrug thereof) that is used for treating, ameliorating, or preventing a diagnosed disease or condition (e.g., skin cancer or precancerous lesions thereof) or exhibits a detectable therapeutic or inhibitory effect. The effect can be detected by any assay methods known in the art. The exact effective amount for a subject depends on: the subject's body weight, physique,

and health status; the nature and severity of the disorder; as well as the therapeutic agent or combination of therapeutic agents selected for administration. The therapeutically effective amount for a given case can be determined through routine experimentation within the skill and judgment of a clinician.

For any compound, the therapeutically effective amount can be estimated in animal models, typically rats, mice, rabbits, dogs, or pigs. Animal models can also be used to determine appropriate concentration ranges and routes of administration. Such information can then be utilized to determine useful doses and routes of administration in humans. Therapeutic/prophylactic efficacy and toxicity can be determined by standard pharmaceutical procedures in cell cultures or experimental animals, such as determining the ED50 (the dose therapeutically effective in 50% of a population) and LD50 (the dose lethal to 50% of a population). The dose ratio between toxicity and therapeutic effect is the therapeutic index, which can be expressed as the LD50/ED50 ratio. The dose may vary within this range, depending on the dosage form employed and the sensitivity of the subject.

The dose and administration are adjusted to provide sufficient levels of the active ingredient or to maintain the desired effect. Factors that may be considered include the severity of the disease state, location of the disease on the subject, overall health status of the subject, age, body weight, and sex of the subject, diet, time and frequency of administration, pharmaceutical combination(s), response sensitivity, and tolerance/response to treatment. Depending on the half-life and clearance rate, 17β-neriifolin or a pharmaceutically acceptable salt, racemate, stereoisomer, enantiomer, diastereomer, tautomer, metabolite, solvate or prodrug thereof may be administered daily, every other day, every three days, every four days, every five days, every six days, weekly, twice weekly, or once every two weeks.

For any of the methods described herein, 17β-neriifolin or a pharmaceutically acceptable salt, racemate, stereoisomer, enantiomer, diastereomer, tautomer, metabolite, solvate or prodrug thereof may be administered topically, intradermally, interepidermally, intragingivally, intraocularly, nasally, ophthalmically, percutaneously, periodontally, subconjunctivally, sublingually, transmucosally, or aurally. In one embodiment, 17β-neriifolin or a pharmaceutically acceptable salt, racemate, stereoisomer, enantiomer, diastereomer, tautomer, metabolite, solvate or prodrug thereof may be administered topically.

Unless otherwise stated, all percentages and ratios used herein are by weight.

The term "17β-neriifolin" as used herein has a molecular formula as follows:

HO OH OH H H H O O O O O

Skin cancer is predominantly caused by prolonged sun exposure, ultraviolet radiation, exposure to biochemical carcinogens, and genetic factors. For example, squamous cell carcinoma tumor cells typically originate from the epidermis and irregularly infiltrate into the dermis as the tumor grows. Therefore, specific delivery to the lesions are required to meet the drug delivery demands of treating the disorders. The inventors of the present application have discovered that 17β-neriifolin or a pharmaceutically acceptable salt, racemate, stereoisomer, enantiomer, diastereomer, tautomer, metabolite, solvate or prodrug thereof has a significant inhibitory effect on skin cancer cells, thus enabling its use in the preparation of a medicament for treating skin cancer.

It is further found that 17β-neriifolin or a pharmaceutically acceptable salt, racemate, stereoisomer, enantiomer, diastereomer, tautomer, metabolite, solvate or prodrug thereof is capable of percutaneous penetration, and when topically administered to skin lesions, it can significantly inhibit tumor cell growth, thereby enabling its formulation to be a medicament suitable for topical use with direct actions on skin lesions. Compared to oral or injectable pharmaceutical formulations, the topical pharmaceutical formulation of 17β-neriifolin can increase the amount of the drug retained in the lesions in the target organ, i.e., the skin, and reduce the amount of the drug entering blood circulation, thereby reducing the systemic adverse effects associated with the drug.

In the medicament, 17β-neriifolin or a pharmaceutically acceptable salt, racemate, stereoisomer, enantiomer, diastereomer, tautomer, metabolite, solvate or prodrug thereof is maintained in a substantially dissolved form. Preferably, the medicament comprises at least 50% of the dissolved form; more preferably, the medicament comprises at least 70% of the dissolved form; more preferably, the medicament comprises at least 90% of the dissolved form; more preferably, the medicament comprises 100% of the dissolved form. Undissolved crystals may be present in any known polymorphic forms. The particle size of 17β-neriifolin or a pharmaceutically acceptable salt, racemate, stereoisomer, enantiomer, diastereomer, tautomer, metabolite, solvate or prodrug thereof may vary from 1 micron to 100 microns (D90) as measured

by laser diffraction.

In some embodiments, the pharmaceutical composition is prepared from 17β-neriifolin or a pharmaceutically acceptable salt, racemate, stereoisomer, enantiomer, diastereomer, tautomer, metabolite, solvate or prodrug thereof, and pharmaceutically acceptable excipients, such that the pharmaceutical composition is a medicament suitable for topical use.

The medicament provided according to the present application may take any suitable form, adopting an appropriate form corresponding to the intended use. By way of example and without limitation, the medicament may be formulated as a powder, spray, ointment, paste, cream, lotion, solution (e.g., rinse, suspension), patch, inhalant, gel (e.g., hydrogel), or emulsion for direct topical application. Depending on the dosage form and use, suitable pharmaceutically acceptable excipients may be selected to formulate a topical formulation in the corresponding form, wherein the pharmaceutically acceptable excipients include: vehicles, diluents, solubilizers, emulsifying agents, antioxidants, binders, dispersants, lubricants, preservatives, and the like.

In some embodiments, the pharmaceutical composition is a gel.

In some embodiments, the pharmaceutically acceptable excipients in the gel optionally include a gel matrix. The gel has a large drug-loading capacity, possesses administration dose flexibility, and can adhere to administration sites for a long time, exhibiting a long-lasting and mild effect. In addition, it demonstrates good biocompatibility and stability, and is easy to apply and clean.

In some embodiments, the gel matrix is selected from at least one of sodium carboxymethyl cellulose, carbomer, gelatin, and alginate. Various types of carbomer can be used, such as Carbomer 934, Carbomer 940, Carbomer 910, or mixtures thereof, with Carbomer 940 being preferred. Carbomer is commercially available, e.g., from BF Goodrich. In some embodiments, the pharmaceutically acceptable excipients in the gel optionally include a humectant. The humectant can increase the adhesion of the formulation, so that the formulation has relatively good spreadability and viscosity.

The penetration enhancer can increase the rate at which the active ingredient penetrates the skin, delivering the active ingredient to the desired site of action in the epidermis and/or dermis. The penetration enhancer can minimize skin irritation and systemic exposure, while achieving local skin/epidermal delivery. Furthermore, it ensures that the majority of the drug substantially remains localized within the skin (i.e., at the site of action) to treat specific skin disorders, while also reducing systemic toxic side effects.

The term "wt%" as used herein refers to the weight of a component relative to the total components, unless otherwise stated.

In the embodiments of the present application, "treat", "treating", or "treatment" refers to obtaining a desired pharmacological and/or physiological effect. The effect may be prophylactic, so as to completely or partially prevent a disease or a symptom thereof; and/or may be therapeutic, so as to partially or completely cure a disease and/or the adverse effects caused by the disease. "Treat", "treating", or "treatment" used herein encompasses a disease in mammals, particularly in humans, including: inhibiting the disease, e.g., blocking the progression of the disease; or alleviating the disease, e.g., mitigating a symptom associated with the disease. "Treat", "treating", or "treatment" as used herein encompasses any administration of a drug or compound to an individual to treat, cure, alleviate, ameliorate, mitigate, or inhibit a disease in the individual, including but not limited to administration of a medicament comprising the drug described herein to an individual in need thereof. For the treatment of skin cancer or precancerous lesions thereof, the pharmaceutical composition of the present application may be administered topically to the affected area(s) in any conventional manner known in the art, for example, by means of a dropper, applicator stick, or cotton swab, by means of intradermal or percutaneous patch delivery, or simply by applying the formulation to the skin area(s) with a finger, sponge, pad, or swab.

The pharmaceutical composition may be used in combination with one or more additional methods and drugs for treating skin cancer or precancerous lesions thereof. The pharmaceutical composition described above may be administered to a subject simultaneously or sequentially with other drugs or treatments within a certain time interval, such that the active ingredients or agents can act cooperatively to treat or prevent skin cancer or precancerous lesions thereof.

The compounds decribed herein contain asymmetric centers and therefore may exist as enantiomers. The compounds have multiple asymmetric centers, which additionally allow for their existence as diastereomers. Enantiomers and diastereomers fall within the broader class of stereoisomers. All such possible stereoisomers include substantially pure resolved enantiomers, and racemic mixtures and diastereomeric mixtures thereof. All stereoisomers of the compounds and/or pharmaceutically acceptable salts thereof are encompassed. Unless otherwise stated, reference to one isomer applies to any possible isomers. When the isomeric components are not specified, all possible isomers are included.

A mixture of diastereomers can be separated into individual diastereomers based on their physicochemical differences by methods well known to those skilled in the art, such as chromatography and/or fractional crystallization. Enantiomers can be separated by converting the enantiomeric mixture into a diastereomeric mixture by reaction with a suitable optically active compound (e.g., a chiral auxiliary such as a chiral alcohol or Mosher's acid chloride), then

separating the diastereomers, and converting (e.g., hydrolyzing) each diastereomer to the corresponding pure enantiomer. Enantiomers can also be separated using a chiral HPLC column.

"Pharmaceutically acceptable salt" includes, but is not limited to: salts of inorganic acids, selected from, for example, hydrochloride, phosphate, hydrogen phosphate, hydrobromide, sulfate, sulfite, and nitrate; and salts of organic salts, selected from, for example, malate, maleate, fumarate, tartrate, succinate, citrate, lactate, methanesulfonate, *p*-toluenesulfonate, 2-hydroxyethylsulfonate, benzoate, salicylate, stearate, alkanoate such as acetate, and salts of HOOC-(CH2)n-COOH, where n is selected from 0-4. Similarly, examples of pharmaceutically acceptable cations include, but are not limited to, sodium, potassium, calcium, aluminum, lithium, and ammonium salts.

Additionally, if the compound is obtained as an acid addition salt, the free base can be obtained by basifying a solution of the acid salt. Conversely, if the product is a free base, an addition salt (e.g., a pharmaceutically acceptable addition salt) can be prepared by dissolving the free base in a suitable organic solvent and treating the solution with an acid, consistent with the conventional procedures for preparing an acid addition salt from a basic compound. Those skilled in the art will appreciate a variety of synthetic methods that can be used to prepare non-toxic pharmaceutically acceptable addition salts without undue experimentation.

"Prodrug" refers to a compound that is converted into the parent drug *in vivo*. Prodrugs are often useful because, in some cases, they may be easier to administer than the parent drug. They may be bioavailable, for example, by oral administration, whereas the parent drug is not. The prodrug may also exhibit improved solubility in a pharmaceutical composition compared to the parent drug. An example (without limitation) of the prodrug is a compound as described herein which is administered as an ester (the "prodrug") to facilitate transport across the cell membrane where water solubility is disadvantageous for this transportation, but which is subsequently metabolically hydrolyzed to a carboxylic acid and an active entity, once inside the cells where water solubility becomes advantageous. A further example of the prodrug may be a short peptide (polyamino acid) linked to an acid group, wherein the peptide can be metabolized to release the active moiety. In certain embodiments, when administered *in vivo*, the prodrug is chemically converted into the biologically, pharmacologically, or therapeutically active form of the compound. In certain embodiments, the prodrug is enzymatically metabolized through one or more steps or processes to yield the biologically, pharmacologically, or therapeutically active form of the compound. To produce a prodrug, a pharmaceutically active compound is modified such that the active compound is regenerated upon *in vivo* administration. The prodrug may be designed to alter the drug's metabolic stability or transport properties to mask side effects or toxicity, thereby improving the effect of the drug or altering other properties or characteristics of the drug. Based on the knowledge of pharmacodynamic methods and *in vivo* drug metabolism, once a pharmaceutically active compound is known, a person skilled in the art can design prodrugs of the compound.

"Metabolite" of the compound disclosed herein is a derivative of the compound that is formed when the compound is metabolized. The term "active metabolite" refers to a biologically active derivative of the compound that is formed when the compound is metabolized. The term "metabolized" as used herein refers to the collective processes by which a particular substance is altered within an organism (including, but not limited to, hydrolysis reactions and enzyme-catalyzed reactions such as oxidation reactions). Thus, enzymes can result in specific structural transformations into new compounds. For example, cytochrome P450 catalyzes various oxidation and reduction reactions, while diphosphoglucuronosyltransferases catalyze the conversion of activated glucuronic acid molecules to aromatic alcohols, aliphatic alcohols, carboxylic acids, amines, and free sulfhydryl groups.

Although specific embodiments of the present disclosure have been illustrated and described, various other changes and modifications may be made without departing from the spirit and scope of the present disclosure. The scope of the appended claims encompasses all such changes and modifications that fall within the scope of the present disclosure.

Other features and advantages of the present application will be apparent from the various embodiments. The provided embodiments are illustrative of various components and methodologies useful in implementing the present application. These embodiments do not limit the claimed application. Based on the present disclosure, a skilled artisan can identify and employ other components and methodologies that can be used to implement the present application.

The additional aspects and the advantages of the present application will be partially provided in the following descriptions, will partially become apparent from the following description, or will be learned through the practice of the present application.

## BRIEF DESCRIPTION OF THE DRAWINGS

Upon reading the detailed description of the non-limiting embodiments made with reference to the following drawings, other features and the objectives and advantages of the present application will become more apparent.

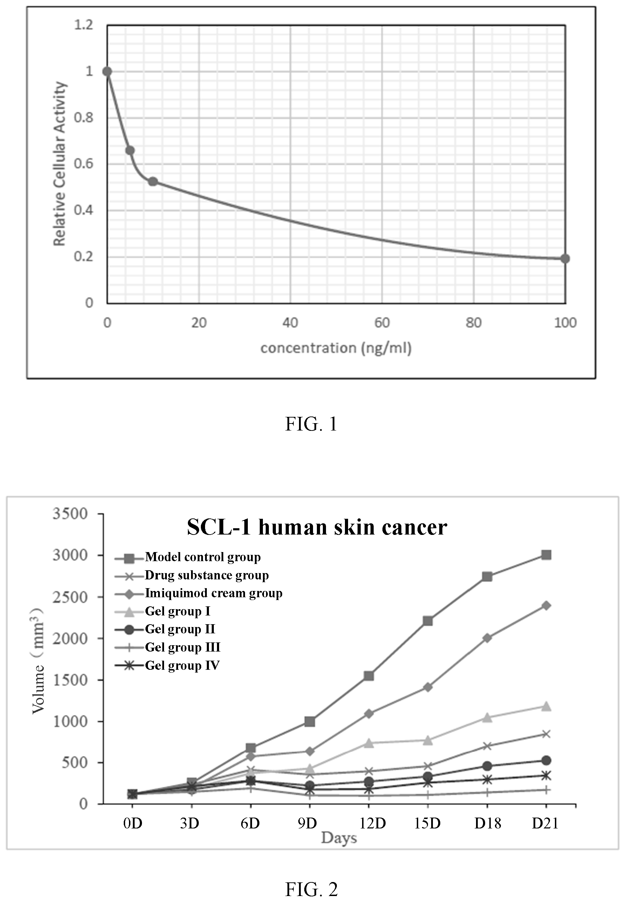

FIG. 1 shows the effects of different concentrations of 17β-neriifolin on the viability of TE.354.T cells (human cutaneous basal cell carcinoma).

FIG. 2 shows the effects of different drugs on the tumor volume in SCL-1 cell (a human cutaneous squamous cell carcinoma cell line) tumor-bearing nude mice, where the experimental groups included: a model control group, an Imiquimod cream group (50 mg/g), a 17β-neriifolin drug substance group (1.6 mg/kg; intragastric(i.g.) administration, once every other day), and 17β-neriifolin groups I, II, III (2 mg/g, 4 mg/g, and 8 mg/g, respectively; administration on the skin at the tumor sites, once every other day), and group IV (4 mg/g; administration on the skin at the tumor sites, once daily); all groups of mice were subjected to administration for three consecutive weeks.

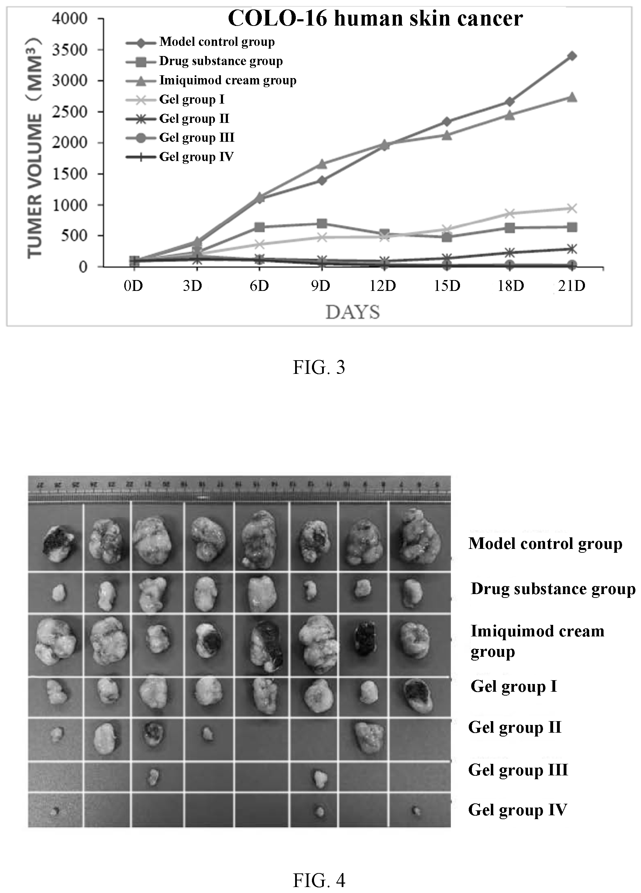

FIG. 3 shows the effects of different drugs on the tumor volume in COLO-16 cell (a human cutaneous squamous cell carcinoma cell line) tumor-bearing nude mice, where the specific groups included: a model control group, an Imiquimod cream group (50 mg/g), a 17β-neriifolin drug substance group (0.8 mg/kg; intragastric(i.g.) administration, once every other day), and 17β-neriifolin groups I, II, III (2 mg/g, 4 mg/g, and 8 mg/g, respectively; administration on the skin at the tumor sites, once every other day), and group IV (4 mg/g; administration on the skin at the tumor sites, once daily); all groups of mice were subjected to administration for three consecutive weeks.

FIG. 4 shows the effects of different drugs on the tumor size in COLO-16 cell (a human cutaneous squamous cell carcinoma cell line) tumor-bearing nude mice, where the specific groups included: a model control group, an Imiquimod cream group (50 mg/g), a 17β-neriifolin drug substance group (0.8 mg/kg; intragastric(i.g.) administration, once every other day), and 17β-neriifolin groups I, II, III (2 mg/g, 4 mg/g, and 8 mg/g, respectively; administration on the skin at the tumor sites, once every other day), and group IV (4 mg/g; administration on the skin at the tumor sites, once daily); all groups of mice were subjected to administration for three consecutive weeks.

## DETAILED DESCRIPTION

The present application will be further illustrated in detail with reference to the following examples. It should be understood that the specific embodiments described herein are merely intended to illustrate, rather than limit, the present disclosure.

It should be noted that the examples and features in the embodiments of the present application may be combined with one another in the absence of conflict.

It should be noted that the endpoints of the ranges and any values disclosed herein are not limited to the precise ranges or values, and these ranges or values should be construed as encompassing values that are close to these ranges or values. The numerical ranges between the endpoint values of each range, between an endpoint value and a single point value of each range, and between two single point values, can be combined with one another to obtain one or more new numerical ranges, which should be construed as being specifically disclosed herein.

The examples without specified techniques or conditions were conducted according to the techniques or conditions described in the literature of the art or according to product instructions. The adopted reagents or instruments without specified manufacturers are all conventional, commercially available products.

Example 1

In this example, the growth inhibitory effects of 17β-neriifolin on TE.354.T, A431, SCL-1, and COLO-16 cells were investigated. The experimental results showed that the compound exhibited strong growth inhibitory effects on these cancer cells. The experimental procedures were as follows:
The cells were subcultured in a complete medium containing 10% FBS in an incubator at 37 °C with 5% $CO_2$ and saturated humidity. The cell density was adjusted to (3-5) $\times 10^4$ cells/mL using a complete medium containing 10% FBS. The cell suspension was added to the wells of a 96-well culture plate at 100 μL/well, and the plate was incubated in an incubator at 37 °C with 5% $CO_2$ and saturated humidity overnight (for about 16-24 hrs). Various dose groups of the test substance and a solvent control group were set. On the next day, the original culture medium was discarded. Subsequently, 20% FBS-containing complete medium was added at 100 μL/well, and the test substance or solvent in FBS-free medium was then added at 100 μL/well, resulting in a final FBS concentration of 10%. Meanwhile, a negative control group was set: each well received 100 μL of 20% FBS-containing complete medium + 100 μL of incomplete medium containing cells, resulting in a final FBS concentration of 10%. A zero adjustment group (background) was also set: 200 μL of the culture medium alone (100 μL of 20% FBS-containing complete medium + 100 μL of incomplete medium), without drugs and cells. For each concentration group, four replicate wells were set.

After incubation for 48 hrs under the experimental conditions, the supernatant was discarded, and 110 μL of CCK-8 working solution (100 μL of 10% FBS-containing complete medium and 10 μL of CCK-8 solution) were added to each well. The cells were further incubated for 4 hrs, and the optical density value at a wavelength of 450 nm (A450) was then measured for each well using a microplate reader. The average value for the wells of each treatment group (T) was compared with the average value for the negative control wells (C). The percent inhibition rate of each concentration group was calculated using the following formula: [Inhibition rate = (1 - T/C) $\times$ 100%], and the half-maximal inhibitory concentration ($IC_{50}$) was calculated. The specific experimental results are shown in the table below:

| Cell | | | Concentration (ng/mL) | | | | | | | $IC_{50}$ |
|---|---|---|---|---|---|---|---|---|---|---|
| | | | 100 | 50 | 25 | 10 | 5 | 2.5 | 1 | |
| COLO-16 | Inhibition rate% | Test 1 | 85.3 | 74.5 | 57.3 | 5.9 | -1.6 | 9.0 | 18.9 | 18.60 |
| | | Test 2 | 89.5 | 77.9 | 72.7 | 45.7 | 31.1 | 6.9 | 5.3 | 12.28 |

TE.354.T: TE.354.T cells were taken and subcultured in a complete medium containing 15% FBS in an incubator at 37 °C with 5% $CO_2$ and saturated humidity. The cell density was adjusted to 1 x $10^4$ cells/mL using a complete medium containing 15% FBS. The cell suspension was added to the wells of a 24-well culture plate at 1 mL/well, and the plate was incubated in an incubator at 37 °C with 5% $CO_2$ and saturated humidity overnight (for about 16-24 hrs). Various dose groups of the test substance, a positive drug Cisplatin group, and a solvent control group were set. On the next day, 1 μL/well of the test substance at different concentrations was added, and 2 μL/well of the positive drug Cisplatin at 5 mg/mL were added, respectively. The cells were cultured under the experimental conditions, and photographed daily to record their growth and survival status. After 72 hrs, the supernatant was discarded, and 0.5 mL of CCK8 working solution (containing 50 μL of CCK8 stock solution) was added to each well. The plate was left to stand in a cell incubator for 2-3 hrs, and the optical density value at a wavelength of 450 nm (A450) was then measured for each well using a microplate reader. The value for the wells of each treatment group (T) was compared with the value for the negative control wells (C). The percent inhibition rate of each concentration group was calculated using the following formula: [Inhibition rate = (1 - T/C) × 100%], and the half-maximal inhibitory concentration (IC50) was calculated. The results, as shown in FIG. 1, indicate that the half-maximal inhibitory concentration was about 10 ng/mL, demonstrating a very high activity in inhibiting cancer cell proliferation.

Example 2

17β-Neriifolin is poorly soluble in water, with a water solubility of about 70 μg/mL. Therefore, the solubility of 17β-neriifolin in organic solvents at different concentrations was investigated. The experimental results are shown in the table below:

| Solvent | Saturation solubility (mg/mL) |
|---|---|
| 10% ethanol | 0.16 |
| 20% ethanol | 0.38 |
| 30% ethanol | 0.91 |
| 40% ethanol | 2.17 |
| 10% propylene glycol | 0.08 |
| 20% propylene glycol | 0.10 |
| 30% propylene glycol | 0.42 |
| 40% propylene glycol | 0.75 |

It can be seen that 17β-neriifolin demonstrated relatively good solubility in ethanol and propylene glycol. Therefore, ethanol and propylene glycol can be used as solvents to dissolve 17β-neriifolin.

Example 3

The solubility of 17β-neriifolin in ethanol at different concentrations was studied (at 20 °C).

An appropriate amount of 17β-neriifolin drug substance was weighed and divided into 3 portions, each placed in a beaker. The first portion was dissolved in anhydrous ethanol and then diluted with water to achieve a final ethanol concentration of 20% and a sample concentration of 8 mg/g, and the diluted solution was observed for the presence of precipitation. The second portion was dissolved in anhydrous ethanol and then diluted with water to achieve final ethanol concentrations of 75%, 66%, 50%, and 40%, corresponding to sample concentrations of 50 mg/g, 40 mg/g, 32 mg/g, and 24 mg/g, respectively, and the diluted solutions were observed for the presence of precipitation. The third portion was dissolved in anhydrous ethanol and then diluted with water to achieve an ethanol concentration of 45% and a sample concentration of 20 mg/g, and the diluted solution was observed for the presence of precipitation. The results are shown in

the table below:

| No. | 17β-Neriifolin (mg) | Anhydrous ethanol (g) | Water (g) | Total solvent amount (g) | Ethanol concentration (%) | Sample concentration (mg/g) | Dissolution behavior |
|---|---|---|---|---|---|---|---|
| 1 | 81 | 2.00 | 8.00 | 10 | 20 | 8 | Dissolved |
| 2 | 201 | 3.98 | 0 | 3.98 | 100 | 50 | Dissolved |
| | | 3.98 | 1.00 | 4.98 | 75 | 40 | Dissolved |
| | | 3.98 | 2.00 | 5.98 | 66 | 32 | Dissolved |
| | | 3.98 | 4.00 | 7.98 | 50 | 24 | Dissolved |
| 3 | 202 | 4.50 | 5.50 | 10.00 | 45 | 20 | Dissolved |

It can be seen that 17β-neriifolin can be dissolved in 20% ethanol at 8 mg/g, in 45% ethanol at 20 mg/g,

Example 4

Compatibility test of sodium carboxymethyl cellulose and Carbomer with 17β-neriifolin:
17β-Neriifolin was mixed with sodium carboxymethyl cellulose or Carbomer in a mass ratio of 1:5, and the mixtures were stored for 10 days under the conditions of 60 °C±2 °C, RH 90%±5%, and a total cumulative illuminance of greater than 1.2 x 106 Lux•hr to investigate the impact of the gel matrix on the stability of 17β-neriifolin. The experimental results show that the high temperature, high humidity, and illumination exhibited no impact on the related substances and drug content of 17β-neriifolin combined with sodium carboxymethyl cellulose, and the stability of this combination was significantly superior to that of the combination with Carbomer.

The detailed results of the compatibility test of 17β-neriifolin with sodium carboxymethyl cellulose are shown in the table below.

| | Condition | Labeled amount (%) | Related substances (%) | | | | | | | | | |
|---|---|---|---|---|---|---|---|---|---|---|---|---|
| | | | Impurity D | Impurity E | Impurity A | Impurity B | RRT0.73 | Impurity C | RRT1.10 | Impurity F | RRT2.18 | Total impurities |
| CMC-Na | 0 days | 99.56 | 0.13 | 0.15 | 0.17 | 0.36 | 0.05 | 0.25 | 0.04 | 0.14 | 0.03 | 1.32 |
| | High temperature for 10 days | 100.95 | 0.13 | 0.15 | 0.17 | 0.36 | 0.05 | 0.25 | 0.04 | 0.14 | 0.03 | 1.32 |
| | High humidity for 10 days | 99.42 | 0.13 | 0.15 | 0.17 | 0.36 | 0.05 | 0.25 | 0.04 | 0.14 | 0.03 | 1.32 |
| | Strong light for 10 days | 99.96 | 0.13 | 0.15 | 0.17 | 0.36 | 0.05 | 0.25 | 0.04 | 0.14 | 0.03 | 1.32 |

Example 5

Study on the effects of different gel matrix contents on the quality of the gel of the present application: 17β-Neriifolin drug substance was weighed and dissolved in anhydrous ethanol aided by ultrasonication and stirring. Azone was added, and the mixture was stirred until uniformity. Water was added, and the mixture was stirred until uniformity. Sodium carboxymethyl cellulose was added, and the mixture was stirred until uniformity. Glycerol was added, and the mixture was stirred until uniformity and allowed to swell overnight. The mixture was then homogenized to obtain the finished gel product. The physical properties of each gel were scored, and the experimental results are shown in the table below:

| Composition | Formula 1 (%) (%) (%) (%) | Formula 2 (%) | Formula 3 (%) | Formula 4 (%) |
|---|---|---|---|---|
| 17β-Neriifolin | 0.4 | 0.4 | 0.4 | 0.4 |
| Azone | 1.0 | 1.0 | 1.0 | 1.0 |
| Anhydrous ethanol | 40 | 40 | 40 | 40 |
| Glycerol | 20 | 20 | 20 | 20 |
| Sodium carboxymethyl cellulose | 1.0 | 1.5 | 2.0 | 3.0 |
| Making up with water to | 100 | 100 | 100 | 100 |
| Physical property score | 16 | 18 | 17 | 17 |

A total score within the range of 1-10 indicates that the prepared gel has poor physical properties, which may affect medication experience of patients; a total score within the range of 11-15 indicates that the gel is suitable for topical administration with relatively good medication experience; and a total score within the range of 16-20 indicates that the gel possesses good physical properties and is easy to administer without causing any discomfort during administration. It can be seen that when the content of sodium carboxymethyl cellulose is in the range of 1.0-3.0 wt%, the resulting gels have good physical properties.

Example 6

Gel formulations were prepared according to the method in Example 5 to study the impact of different humectant contents on the quality of the gel of the present application. The experimental results are shown in the table below:

| Composition | Formula 5 (%) | Formula 6 (%) | Formula 7 (%) |
|---|---|---|---|
| 17β-Neriifolin | 0.4 | 0.4 | 0.4 |
| Azone | 1.0 | 1.0 | 1.0 |
| Anhydrous ethanol | 40 | 40 | 40 |
| Glycerol | 15 | 20 | 25 |
| CMC-Na | 1.5 | 1.5 | 1.5 |
| Making up with water to | 100 | 100 | 100 |
| Physical property total score | 18 | 18 | 17 |

It can be seen that when the content of glycerol is in the range of 15-25 wt%, the resulting gels have good physical properties.

Example 7

Preparation of the gel:

| 17β-Neriifolin | Ethylparaben | Glycerol | Ethanol | CMC-Na | Making up with water to |
|---|---|---|---|---|---|
| 105.80mg | 0.25g | 12.5ml | 12ml | 0.5g | 50g |

Distilled water (25 mL) and glycerol (12.5 mL) were mixed until uniformity, and 17β-neriifolin and ethanol (10 mL) were added. The mixture was ultrasonicated to aid dissolution.

The formula amount of ethylparaben was then dissolved in ethanol (2 mL) with stirring until uniformity, and the mixture was added to the above solution. The resulting mixed solution was stirred until uniformity.

The formula amount of CMC-Na was added to the above mixed solution, and the mixture was stirred until a homogeneous state was achieved. Water was then added to adjust the total mass to 50 g, and the resulting mixture was stirred until uniformity to obtain the final gel.

Example 8

Gels were prepared according to the method in Example 5 to study the skin irritation caused by the gels containing different penetration enhancers and different amounts of the penetration enhancers:
Healthy young pigs (half male and half female) with no back skin injury were used. Comparative administration was performed by topical application on the left and right sides of the same animal's skin, and the administration sites were covered with patches after application. The test gel was administered to the left side, and white vaseline was administered to the right side as a negative control. The administration area was 3.0 cm × 3.0 cm, and the dose was 20 mg/cm$^2$. The administration was performed once daily. The patches on the administration sites were maintained on both sides of the skin for about 4 hrs before removal, and the administration sites were then cleaned with a warm 0.9% sodium chloride injection solution. A single dose was administered. Potential erythema and edema at the administration sites were examined and recorded for each group of animals prior to administration and at 1 h, 24 hrs, 48 hrs, 72 hrs and 14 days after drug removal. Erythema and edema were recorded and scored according to the scoring criteria. The experiment revealed that all the test gels met the irritation criteria.

Each test gel contained 1.2 wt% 17β-neriifolin, 1.5 wt% sodium carboxymethyl cellulose, 40 wt% anhydrous ethanol, 0.5 wt% to 1.0 wt% penetration enhancer, and 20 wt% glycerol, with the balance being water. The 0.5 wt% to 1.0 wt% penetration enhancers were 1.0% azone, 1.0% borneol, 0.75% borneol, and 0.5% borneol, respectively.

Example 9

The Franz diffusion cell method was used to determine the *in vitro* transdermal absorption, including the transdermal amount, residual amount on the skin surface, and intradermal retention amount.

Transdermal amount: Pig skin (which had been immersed in normal saline for about 30 min before use, and was then taken out and dried with filter paper) was fixed between the receiving cell and dosing cell, with the stratum corneum facing the dosing cell. About 200 mg of the gel of the present application were precisely weighed and evenly applied to the pig skin on the dosing cell side. Eighteen milliliters of 15% ethanol-normal saline preheated to 32 °C±0.5 °C were added to the receiving cell and stirred at a rotation speed of 200 rpm. Then, 0.5 mL of the receiving solution was sampled at 1 h, 2 hrs, 3 hrs, 4 hrs, and 6 hrs (with an equal volume of a blank receiving solution at the same temperature added into the receiving cell at the same time). The samples were centrifuged, and the supernatants were collected and subjected to HPLC analysis. The transdermal amount of the drug was calculated based on the peak area.

Residual amount on the skin surface: After permeation with the formulation of the present application for 6 hrs, the pig skin (ensuring that the gel remaining on the surface was not lost) was rinsed with a total of 25 mL of a saturated sodium chloride solution (5 mL each time for 5 times, vortexed for 0.5 min per wash). The rinsates were collected into a 100-mL volumetric flask, and 25 mL of methanol were added. The mixture was ultrasonicated for extraction for 20 min, diluted to the volume with a 50% methanol-saturated sodium chloride solution, mixed by shaking until uniformity, and then filtered. The subsequent filtrate was collected and subjected to HPLC analysis, and the residual amount on the skin surface was calculated based on the peak area.

Intradermal retention amount: The rinsed pig skin was treated with 25 mL of an 80% methanol-saturated sodium chloride solution, heated in a water bath at 70 °C for 30 min, cut into pieces, and then homogenized. The homogenate was centrifuged (at 4 °C and 14000 rpm for 10 min). The supernatant was collected and filtered. The subsequent filtrate was collected and subjected to HPLC analysis, and the intradermal retention amount was calculated based on the peak area.

The test gel was prepared according to the method in Example 5, containing 0.4 wt% 17β-neriifolin, 1.5 wt% sodium carboxymethyl cellulose, 40 wt% anhydrous ethanol, 0.5 wt% borneol, and 20 wt% glycerol, with the balance being water.

It was found through calculation that within 6 hrs, the drug in the gel of the present application had a transdermal

amount of less than 1%, a residual amount on the skin surface of 80%-95%, and an intradermal retention amount of approximately 10%. The results suggest that the drug in the gel prepared by the present disclosure should be substantially retained with the skin tissue, achieving a high local drug concentration, and little would be absorbed into the blood, so that the side effects caused by systemic drug distribution can be reduced.

Example 10

Healthy SPF-grade Hartley guinea pigs (half male and half female) with no back skin injury were selected. A negative control group (white vaseline), a positive control group (2,4-dinitrochlorobenzene vaseline ointment), test substance group I (17β-neriifolin gel, with the content of 17β-neriifolin being 2 mg/g), test substance group II (17β-neriifolin gel, with the content of 17β-neriifolin being 4 mg/g), and test substance group III (17β-neriifolin gel, with the content of 17β-neriifolin being 8 mg/g) were set. For all groups, the dose was 0.5 g of gel/animal/administration, and the administration area on the skin was 2.5 cm × 2.5 cm. The drug in each group was kept in contact with the left dorsal skin of each animal in the corresponding group for 6 hrs to test the sensitivity. Potential skin reactions were observed, recorded, and scored prior to administration and at 1 h and 24 hrs after residual drug removal. The drug administration for sensitivity testing was conducted once on day 1, day 8, and day 15, for a total of three applications. On day 29, the drug was kept in contact with the right-side skin of the animals for 6 hrs. Potential skin reactions were observed and recorded prior to administration and at 24 hrs and 48 hrs after residual drug removal. The test results were assessed.

The test gels of the test substance groups I, II, and III were prepared according to the method in Example 5, each containing 1.5 wt% sodium carboxymethyl cellulose, 40 wt% anhydrous ethanol, 0.5 wt% borneol, and 20 wt% glycerol, with the balance being water.

The results showed that the gel formulations of the present application did not cause allergic reactions in the skin of guinea pigs, with all allergic reaction results being negative.

Example 11

Healthy New Zealand rabbits (half male and half female) with no back injury were used. A blank gel control group and 17β-neriifolin gel groups (with the contents of 17β-neriifolin being 2 mg/g and 4 mg/g, respectively) were set. Comparative administration was performed by topical application on the left and right sides of the same animal's skin, and the administration sites were covered with patches after application. The test substance was administered to the left side, and white vaseline was administered to the right side as a negative control. The administration area was 3.0 cm × 3.0 cm. The patches on the administration sites were maintained on both sides of the skin for about 4 hrs before removal, and the administration sites were then cleaned with a warm 0.9% sodium chloride injection solution. A single dose was administered. Potential erythema and edema at the administration sites were examined and recorded for each group of animals prior to administration and at 1 h, 24 hrs, 48 hrs, 72 hrs and 14 days after drug removal. The erythema and edema were recorded and scored according to the scoring criteria. Additionally, the left-side skin of the administration sites in each group of animals was subjected to histopathological examination at 72 hrs and 14 days after drug removal.

The results show that after single-dose topical administration on the intact skin of New Zealand rabbits in the blank gel control group and the 17β-neriifolin gel groups (2 mg/g and 4 mg/g), *both left and right skin sites of each animal exhibited irritation reaction scores within the non-irritating* range, with no abnormalities found in microscopic examination and no test substance-related skin irritation reactions observed.

The test gel was prepared according to the method in Example 5, containing 1.5 wt% sodium carboxymethyl cellulose, 40 wt% anhydrous ethanol, 0.5 wt% borneol, and 20 wt% glycerol, with the balance being water.

Example 12

Quarantine-qualified male BALB/c-nu nude mice weighing 13.4-20.5 g were each inoculated with 0.2 mL of SCL-1 cells in logarithmic growth phase at 3 x $10^6$ cells/mL (with Matrigel and cell suspension mixed in a ratio of 1:2) in the right axilla. When the average tumor volume of the nude mice reached approximately 100 $mm^3$, 84 tumor-bearing nude mice were selected and randomly divided into the following seven groups based on tumor volume (with intact tumors and no ulceration): a model control group, an Imiquimod cream group (50 mg/g), a 17β-neriifolin drug substance group (1.6 mg/kg; intragastric administration, once every other day), and 17β-neriifolin gel groups I, II, III (2 mg/g, 4 mg/g, and 8 mg/g, respectively; administration on the skin at the tumor sites, once every other day) and group IV (4 mg/g; administration on the skin at the tumor sites, once daily). The mice in the model control group, the Imiquimod cream group, and the 17β-neriifolin gel groups I, II, III and IV were each subjected to administration via topical skin application at 0.01 g of gel or cream/$cm^2$ at the tumor-forming sites. The drug was kept in contact with the skin for 4 hrs per administration, and the treatment was given to each group for three consecutive weeks. During the treatment period, the long diameter (a) and short diameter (b) of the tumors were measured using a vernier caliper every 3 days. Then, the tumor volume (TV), relative

tumor volume (RTV), and relative tumor proliferation rate (T/C, %) were calculated. After the final administration, the tumor tissues were dissected out and weighed to calculate the tumor growth inhibition rates.

The gels in 17β-neriifolin gel groups I, II, III and IV were prepared according to the method in Example 5, each containing 1.5 wt% sodium carboxymethyl cellulose, 40 wt% anhydrous ethanol, 0.5 wt% borneol, 20 wt% glycerol, and the corresponding amount of 17β-neriifolin, with the balance being water.

The experimental results are shown in FIG. 2. It can be seen that after 21 days of treatment, the 17β-neriifolin drug substance (1.6 mg/kg) achieved a tumor inhibition rate of greater than 70%, and the tumor inhibition rates in the 17β-neriifolin gel groups I, II, III and IV were greater than 60%, 80%, 95%, and 85%, respectively, indicating that 17β-neriifolin had a remarkable anti-tumor effect, which was significantly superior to that of Imiquimod cream. Therefore, 17β-neriifolin is suitable for formulation into topical medicaments to act on skin lesions for the treatment of skin cancer, improving the therapeutic effect while reducing drug-induced systemic adverse effects.

Example 13

Quarantine-qualified male BALB/c-nu nude mice weighing 13.7-21.1 g were each inoculated with 0.2 mL of COLO-16 cells in logarithmic growth phase at $6.7 \times 10^6$ cells/mL in the right axilla. When the average tumor volume of the nude mice reached approximately 100 $mm^3$, 56 tumor-bearing nude mice were selected and randomly divided into the following seven groups based on tumor volume (with intact tumors and no ulceration): a model control group, an Imiquimod cream group (50 mg/g), a 17β-neriifolin drug substance group (0.8 mg/kg; intragastric administration, once every other day), and 17β-neriifolin gel groups I, II, III (2 mg/g, 4 mg/g, and 8 mg/g, respectively; administration on the skin at the tumor sites, once every other day), and group IV (4 mg/g; administration on the skin at the tumor sites, once daily). The mice in the model control group, the Imiquimod cream group, and the 17β-neriifolin gel groups I, II, III and IV were each subjected to administration via topical skin application at 0.01 g of gel or cream/$cm^2$ at the tumor-forming sites. The drug was kept in contact with the skin for 4 hrs per administration, and the treatment was given to each group for three consecutive weeks. During the treatment period, the long diameter (a) and short diameter (b) of the tumors were measured using a vernier caliper every 3 days. Then, the tumor volume (TV), relative tumor volume (RTV), and relative tumor proliferation rate (T/C, %) were calculated. After the final administration, the tumor tissues were dissected out and weighed to calculate the tumor growth inhibition rates.

The gels in the gel groups I, II, III and IV were prepared according to the method in Example 5, each containing 1.5 wt% sodium carboxymethyl cellulose, 40 wt% anhydrous ethanol, 0.5 wt% borneol, 20 wt% glycerol, and a certain amount of 17β-neriifolin (corresponding to groups I, II, III and IV), with the balance being water.

The experimental results are shown in FIGs. 3-4. It can be seen that after 21 days of treatment, the 17β-neriifolin drug substance (0.8 mg/kg) achieved a tumor inhibition rate of greater than 80%, and the tumor inhibition rates in the 17β-neriifolin gel groups I, II, III and IV were greater than 70%, 90%, 99%, and 99%, respectively, indicating that 17β-neriifolin had a remarkable anti-tumor effect, which was significantly superior to that of Imiquimod cream. Therefore, 17β-neriifolin is suitable for formulation into topical medicaments to act on skin lesions for the treatment of skin cancer, improving the therapeutic effect while reducing drug-induced systemic adverse effects.

Example 14

A375.S2 human malignant melanoma cells were subcutaneously inoculated into the right axilla of nude mice. When the average tumor volume reached approximately 100 $mm^3$, the mice were divided into the following groups: a model control group, a gel group 1 (0.5% borneol), a gel group 2 (1.5% borneol), and a gel group 3 (1.0% azone). Each group contained six animals (all male). The mice were subjected to administration via topical skin application at the tumor-forming sites. The test substances were applied to the corresponding application areas on the animals. The application sites were then covered with a layer of plastic film, followed by two layers of gauze, which were then secured with non-irritating paper adhesive tape. The test substances were administered once daily for two consecutive weeks, with a dose of 0.2 g/$cm^2$ and 6 hrs of contact time per administration. The tumor diameters of the animals were measured, and the relative tumor proliferation rates T/C (%) were calculated. After the nude mice were sacrificed by cervical dislocation, the tumors were dissected out and weighed to calculate the tumor inhibition rates.

The gels in gel groups 1, 2 and 3 were prepared according to the method in Example 5, each containing 0.2 wt% 17β-neriifolin, 1.5 wt% sodium carboxymethyl cellulose, 40 wt% anhydrous ethanol, 20 wt% glycerol, and the corresponding penetration enhancer, with the balance being water.

The experimental results are shown in the table below.

| Group | Dose | | Tumor weight (g) | Tumor inhibition rate (%) | D15 relative tumor volume RTV | D15 T/C (%) |
|---|---|---|---|---|---|---|
| | Based on active ingredient (mg/cm$^2$ tumor area) | mg/g | | | | |
| Model control group | / | / | 1.004±0.165 | / | 6.30±5.99 | / |
| Gel group 1 | 0.4 | 2 | 0.598±0.228* | 40.4 | 3.22±1.54 | 51.0 |
| Gel group 2 | 0.4 | 2 | 0.702±0.305 | 30.1 | 4.20±2.48 | 66.6 |
| Gel group 3 | 0.4 | 2 | 0.486±0.419 | 51.6 | 3.22±3.85 | 51.0 |

It can be seen that the application of 2 mg/g gel containing 0.5% borneol or 1.5% borneol once daily exhibited a trend of anti-tumor effects in the human malignant melanoma cell (A375.S2) tumor-bearing nude mice, and the application of 2 mg/g gel containing 1.0% azone once daily also showed an anti-tumor effect in the human malignant melanoma cell (A375.S2) tumor-bearing nude mice.

The experimental results demonstrated that 17β-neriifolin exhibited higher tumor inhibition rates against human non-melanoma cells (SCL-1 cells and COLO-16 cells) compared to A375.S2 human malignant melanoma cells.

Example 15

Clinical treatment of non-melanoma skin cancer and Bowen's disease with the pharmaceutical composition

Clinical studies were conducted to evaluate the efficacy and safety of 17β-neriifolin topically applied to subjects with non-melanoma skin cancer or Bowen's disease. 17β-Neriifolin showed good clinically relevant efficacy in the treatment of non-melanoma skin cancer and Bowen's disease. From the perspectives of local tolerability and local skin reactions (LSR), 17β-neriifolin was shown to be safe.

This study consisted of Phase I (a dose escalation trial) and Phase II (an expansion trial). Phase I was a multicenter, open-label, and dose-escalation trial investigating the safety, tolerability, PK profile, and preliminary efficacy of 17β-neriifolin gel at different dose levels applied to lesions of Chinese subjects with non-melanoma skin cancers (including basal cell carcinoma, cutaneous squamous epithelial cell carcinoma) and Bowen's disease. Phase II was a multicenter, open-label trial investigating the efficacy, safety and PK profile of 17β-neriifolin gel at the target dose in Chinese subjects with non-melanoma skin cancers (including cutaneous squamous epithelial cell carcinoma and basal cell carcinoma) and Bowen's disease.

1. Case selection

Patients aged ≥ 18 years were selected. All patients complied with the study protocols and provided informed consent.

2. Inclusion criteria

Inclusion criteria: (1) meeting the diagnostic criteria for cutaneous squamous epithelial cell carcinoma, basal cell carcinoma, or Bowen's disease as defined by *Clinical Dermatology*, and being diagnosed via histopathological examination or noninvasive diagnosis (dermatoscopy, skin CT, etc.); and (2) having complete clinical data.

3. Methods

| Dose Group/Level | Dose based on 17β-neriifolin (mg/cm$^2$) | Dose based on gel (mg/cm$^2$) | |
|---|---|---|---|
| | | Dose (mg/cm$^2$) | Gel specification |
| 1 | 0.008 | 4 | 2 mg/g |
| 2 | 0.016 | 8 | 2 mg/g |
| 3 | 0.027 | 6.75 | 4 mg/g |
| 4 | 0.04 | 10 | 4 mg/g |

(continued)

| Dose Group/Level | Dose based on 17β-neriifolin ($mg/cm^2$) | Dose based on gel ($mg/cm^2$) | |
|---|---|---|---|
| | | Dose ($mg/cm^2$) | Gel specification |
| 5 | 0.06 | 15 | 4 mg/g |

The Phase I trial was a dose escalation study involving single-dose and multiple-dose administrations. After single-dose administration, a 3-day DLT (dose-limiting toxicity) observation was performed. Starting on day 4, multiple-dose administration began, with the drug administered once every other day for three consecutive weeks.

Method of administration: Evenly apply the gel to the lesions, avoiding rinsing and rubbing. The application site was then covered with a breathable waterproof dressing and secured with non-irritating adhesive tape. It was ensured that the drug was kept in contact with the skin for 4±0.5 h per administration. To remove the drug, the administration site was rinsed and wiped with room-temperature normal saline. Calculation of administration dose: administration area ($cm^2$) = (radius of affected area)$^2 \times \pi$

Dose (gel, mg) = [dose (gel amount) per $cm^2$ in the current dose group] $\times$ administration area

Conversion formula between mg and microliter: gel weight ÷ gel density = volume (in microliters, μL)

Administration area: 2 treatment units (1 treatment unit was defined as the area of one index finger knuckle of each subject). The total administration area of the target lesions should not exceed 25 $cm^2$.

During the hospitalization period, medication was administered under the supervision of a physician or nurse. The dose and time of administration were recorded in the subject's diary card. During the non-hospitalization period, the study physician and nurse instructed the subjects to apply the medication in a fixed time period each day according to the above-specified administration method. The specific administration regimen is detailed in the standard operating procedures for drug use.

The frequency of administration and intermission periods of the study drug may be appropriately adjusted based on human safety, PK data, and efficacy.

4. Observation indicators

Evaluation of the clinical effects.

The efficacy evaluation indicators for the subject cohorts with cutaneous basal cell carcinoma, cutaneous squamous epithelial cell carcinoma, and Bowen's disease: CR: complete response, indicating the disappearance of all target tumor lesions; PR: partial response, indicating that the tumor volume was reduced by at least 30%; SD: stable disease, indicating no significant change in tumor volume;

Objective response rate (ORR): the proportion of subjects achieving CR or PR (calculated based on the optimal response during the administration period) in the corresponding analysis set;

Disease control rate (DCR): the proportion of subjects achieving CR, PR, or SD (calculated based on the optimal response during the administration period) in the corresponding analysis set;

Duration of response (DOR): the time from the first assessment of CR or PR to the first assessment of PD or death from any cause for a subject;

Time to progression (TTP): the time from the first administration to the date of definite disease progression for a subject;

Progression-free survival (PFS): the time from the first administration to the date of definite disease progression or the date of death from any cause for a subject; Dermatology quality of life index: the SF-36 rating scale was used to evaluate the subject's quality of life;

Safety indicators: adverse events (including local reactions at administration sites), physical examinations, vital signs, laboratory examinations (including blood routine examination, blood biochemistry, urinalysis, etc.), electrocardiograms, etc.

5. **Summary of safety and efficacy data for subjects enrolled in Phase I**

A total of 29 subjects were enrolled in this study. Demographic information of the subjects is shown in the table below.

**Demographic data summary - all enrolled subjects (China and Australia)**

| | 17β-Neriifolin (N = 29) |
|---|---|
| **Age (years)** | |
| n (number of subjects) | 29 |
| Mean (SD) | 72.6 (11.70) |
| Median | 75.0 (67.0,80.0) |
| Min; Max | 32; 91 |
| **Sex, n (%)** | |
| n (number of subjects) | 29 |
| Male | 10 (34.5%) |
| Female | 19 (65.5%) |
| **Ethnicity, n (%)** | |
| n (number of subjects) | 29 |
| Han Chinese | 23 (79.3%) |
| Others | 6 (20.7) |
| **ECOG score, n (%)** | |
| n (number of subjects) | 29 |
| 0 | 27 (93.1%) |
| 1 | 2 (6.9%) |
| 2 | 0 |
| 3 | 0 |
| 4 | 0 |
| 5 | 0 |

**Safety results**

Overall, 29 subjects received at least one application of 17β-neriifolin topical gel. There were five administration dose levels/groups (0.008 mg/cm$^2$, 0.016 mg/cm$^2$, 0.027 mg/cm$^2$, 0.04 mg/cm$^2$, and 0.06 mg/cm$^2$). All groups showed relatively good safety profiles. Regarding the safety profiles, the 17β-neriifolin topical gel showed similar safety profiles in Chinese and Australian subjects. According to the investigators' evaluation, the drug-related adverse events were primarily reactions at the skin application sites.

A total of 20 (69%) subjects experienced treatment-related adverse events (TRAEs) that the investigators assessed as drug-related. The most frequently reported TRAEs were skin pruritus (7/29, 24.1%), skin desquamation (5/29, 17.2%), and skin crusting (5/29, 17.2%). These were mainly mild local reactions at the administration sites. All AEs were Grade 1 or 2 according to the CTCAE, and no Grade 3 or higher TEAEs occurred.

There were no SAEs, and no DLTs were observed.

No fatal AEs were reported.

In summary, the safety profiles of 17β-neriifolin gel were similar in Chinese and Australian subjects. According to the investigators' evaluation, the drug-related adverse events were primarily local reactions at the skin application sites, such as pruritus, desquamation, crusting, and the like, all of which were Grade 1-2 mild reactions, indicating relatively good overall safety.

**Efficacy results:**

Based on the safety, tolerability, pharmacokinetics, and efficacy data generated in this clinical study, the efficacy-

evaluable subjects in the dose groups 1-5 in China and Australia included six cases of cutaneous basal cell carcinoma, one case of *non-in-situ* cutaneous squamous epithelial cell carcinoma, and six cases of Bowen's disease.

**Efficacy of 17β-neriifolin in the treatment of cutaneous basal cell carcinoma**

In dose group 5, a total of six cutaneous basal cell carcinoma patients, who were efficacy-evaluable on D16, on D24, and on day 28 after the last dose during the follow-up, were enrolled. Based on the investigators' assessments according to RECIST1.1 criteria, three subjects achieved a best efficacy assessment result of PR, and the treatment of BCC with 17β-neriifolin gel achieved a disease control rate (DCR) of 100% and an ORR of 50% on D16, on D24, and on day 28 of the follow-up period after the last dose.

**Efficacy of 17β-neriifolin in the treatment of cutaneous squamous cell carcinoma**

In dose group 5, only one patient with cutaneous squamous cell carcinoma was enrolled. This patient was efficacy-evaluable on D16, on D24, and on day 28 of the follow-up period after the last dose. Based on the investigators' assessments according to RECIST1.1 criteria, the subject achieved a best efficacy assessment result of PR on D16, on D24, and on day 28 of the follow-up period after the last dose.

**Efficacy of 17β-neriifolin in the treatment of Bowen's disease**

A total of six Bowen's disease patients who were efficacy-evaluable were enrolled in the dose-escalation phase of this study. Based on the investigators' assessments according to RECIST1.1 criteria, the treatment of Bowen's disease with 17β-neriifolin gel achieved a disease control rate (DCR) of 100%, a CRR (complete response rate) of 16.67%, and an ORR of 66.7%.

**Pharmacokinetic study results:**

Serum concentrations of 17β-neriifolin in the Chinese and Australian patients treated with the topical gel were measured and analyzed during the dose-escalation phase (dose levels 1-5) of the clinical study.

Serum concentrations of 17β-neriifolin were all determined using liquid chromatography-mass spectrometry (LC/MS/MS), with the lower limit of quantification (LLOQ) being 0.02 ng/mL. No dose-dependent increase in the systemic exposure of 17β-neriifolin was observed within a 0.008-0.06 mg/cm$^2$ dose range. Due to the low overall systemic absorption rate and sparse data, a statistically significant pharmacokinetic evaluation could not be performed (only seven Chinese subjects had plasma drug concentrations above the LLOQ and in the concentration range of 0.022-0.106 ng/mL, while a total of four Australian subjects had plasma drug concentrations above the LLOQ and in the concentration range of 0.021-0.101 ng/mL).

It can be seen that the gel of the present application exhibited good therapeutic efficacy and was confirmed to have good safety.

In the description of this specification, the description of reference terms "one embodiment", "some embodiments", "an example", "a specific example", or "some examples", and the like means that a particular feature, structure, material, or characteristic described in connection with the embodiments or examples is included in at least one embodiment or example of the present application. In this specification, the schematic descriptions of the terms described above do not necessarily refer to the same embodiment or example. Furthermore, the specific features, structures, materials, or characteristics described herein may be combined in any suitable manner in any one or more embodiments or examples. In addition, in the absence of contradiction, those skilled in the art can combine the different embodiments or examples described in this specification or combine the features of different embodiments or examples.

Although the examples of the present application have been shown and described above, it will be appreciated that the above examples are exemplary and should not be construed as limiting the present application, and that modifications, substitutions, and alterations can be made to the above examples by those of ordinary skill in the art within the scope of the present application.

## Claims

1. A compound, which is 17β-neriifolin or a pharmaceutically acceptable salt, racemate, stereoisomer, enantiomer, diastereomer, tautomer, metabolite, solvate or prodrug thereof, for use in the treatment and/or prevention of skin cancer or precancerous lesions thereof via topical administration.

2. A method for treating and/or preventing skin cancer or precancerous lesions thereof, comprising administering 17β-neriifolin or a pharmaceutically acceptable salt, racemate, stereoisomer, enantiomer, diastereomer, tautomer, metabolite, solvate or prodrug thereof.

3. The method according to Claim 2, **characterized in that** the method comprises administering to a subject in need thereof a therapeutically effective amount of 17β-neriifolin or a pharmaceutically acceptable salt, racemate, stereoisomer, enantiomer, diastereomer, tautomer, metabolite, solvate or prodrug thereof.

4. The method according to any one of the preceding claims, **characterized in that** the skin cancer comprises melanoma and/or non-melanoma skin cancer.

5. The method according to any one of the preceding claims, **characterized in that** the non-melanoma skin cancer is basal cell carcinoma and/or cutaneous squamous cell carcinoma.

6. The method according to any one of the preceding claims, **characterized in that** the precancerous skin lesions comprise epithelial lesions or abnormal proliferation, preferably being a cancer precursor or potentially becoming a cancer precursor, more preferably being Bowen's disease, arsenical keratosis, radiodermatitis, leukoplakia, or junctional nevus.

7. The method according to any one of the preceding claims, **characterized in that** 17β-neriifolin or a pharmaceutically acceptable salt, racemate, stereoisomer, enantiomer, diastereomer, tautomer, metabolite, solvate or prodrug thereof is administered to the affected area(s) of the subject at a dose of: about 0.0003 mg to about 100 mg; about 0.008 mg to about 90 mg; about 0.1 mg to about 80 mg; about 0.1 mg to about 70 mg; about 0.1 mg to about 60 mg; about 0.1 mg to about 50 mg; about 0.1 mg to about 40 mg; about 0.1 mg to about 30 mg; about 0.1 mg to about 20 mg; about 0.1 mg to about 15 mg; about 0.1 mg to about 10 mg; about 0.1 mg to about 8 mg; about 0.2 mg to about 5 mg; about 0.5 mg to about 2.5 mg; about 0.02 mg, about 0.05 mg, about 0.1 mg, about 0.2 mg, about 0.3 mg, about 0.4 mg, about 0.5 mg, about 0.6 mg, about 0.7 mg, about 0.8 mg, about 0.9 mg, about 1 mg, about 1.1 mg, about 1.2 mg, about 1.3 mg, about 1.4 mg, about 1.5 mg, about 1.6 mg, about 1.7 mg, about 1.8 mg, about 1.9 mg, about 2.0 mg, about 2.1 mg, about 2.2 mg, about 2.3 mg, about 2.4 mg, about 2.5 mg, about 2.6 mg, about 2.7 mg, about 2.8 mg, about 2.9 mg, about 3 mg, about 4 mg, or about 5 mg.

8. The method according to any one of the preceding claims, **characterized in that** 17β-neriifolin or a pharmaceutically acceptable salt, racemate, stereoisomer, enantiomer, diastereomer, tautomer, metabolite, solvate or prodrug thereof is administered to the affected area(s) of the subject at a dose of: about 0.0003 mg/cm$^2$ to about 10 mg/cm$^2$; preferably about 0.001 mg/cm$^2$ to about 1 mg/cm$^2$; preferably about 0.002 mg/cm$^2$ to about 0.5 mg/cm$^2$; preferably about 0.004 mg/cm$^2$ to about 0.25 mg/cm$^2$; preferably about 0.008 mg/cm$^2$ to about 0.2 mg/cm$^2$; preferably about 0.01 mg/cm$^2$ to about 0.18 mg/cm$^2$; preferably about 0.016 mg/cm$^2$ to about 0.16 mg/cm$^2$; preferably about 0.02 mg/cm$^2$ to about 0.15 mg/cm$^2$; preferably about 0.025 mg/cm$^2$ to about 0.14 mg/cm$^2$; preferably about 0.027 mg/cm$^2$ to about 0.13 mg/cm$^2$; preferably about 0.03 mg/cm$^2$ to about 0.125 mg/cm$^2$; preferably about 0.035 mg/cm$^2$ to about 0.12 mg/cm$^2$; preferably about 0.04 mg/cm$^2$ to about 0.1 mg/cm$^2$; about 0.001 mg/cm$^2$, about 0.002 mg/cm$^2$, about 0.003 mg/cm$^2$, about 0.004 mg/cm$^2$, about 0.005 mg/cm$^2$, about 0.006 mg/cm$^2$, about 0.007 mg/cm$^2$, about 0.008 mg/cm$^2$, about 0.009 mg/cm$^2$, about 0.01 mg/cm$^2$, about 0.015 mg/cm$^2$, about 0.02 mg/cm$^2$, about 0.025 mg/cm$^2$, about 0.03 mg/cm$^2$, about 0.035 mg/cm$^2$, about 0.04 mg/cm$^2$, about 0.045 mg/cm$^2$, about 0.05 mg/cm$^2$, about 0.055 mg/cm$^2$, about 0.06 mg/cm$^2$, about 0.065 mg/cm$^2$, about 0.07 mg/cm$^2$, about 0.075 mg/cm$^2$, about 0.08 mg/cm$^2$, about 0.085 mg/cm$^2$, about 0.09 mg/cm$^2$, about 0.095 mg/cm$^2$, about 0.1 mg/cm$^2$, about 0.15 mg/cm$^2$, about 0.2 mg/cm$^2$, about 0.25 mg/cm$^2$, about 0.3 mg/cm$^2$, about 0.35 mg/cm$^2$, about 0.4 mg/cm$^2$, about 0.45 mg/cm$^2$, about 0.5 mg/cm$^2$, about 0.55 mg/cm$^2$, about 0.6 mg/cm$^2$, or about 0.65 mg/cm$^2$.

9. The method according to any one of the preceding claims, **characterized in that** the affected area(s) of the subject is/are about 0.01 cm$^2$ to about 300 cm$^2$; preferably, the affected area(s) of the subject is/are: about 1 cm$^2$ to about 200 cm$^2$, about 1 cm$^2$ to about 100 cm$^2$, about 1 cm$^2$ to about 75 cm$^2$, about 1 cm$^2$ to about 50 cm$^2$, or about 1 cm$^2$ to about 25 cm$^2$; about 10 cm$^2$ to about 200 cm$^2$, about 10 cm$^2$ to about 100 cm$^2$, about 10 cm$^2$ to about 75 cm$^2$, about 10 cm$^2$ to about 50 cm$^2$, or about 10 cm$^2$ to about 25 cm$^2$; about 25 cm$^2$ to about 200 cm$^2$, about 25 cm$^2$ to about 100 cm$^2$, or about 25 cm$^2$ to about 75 cm$^2$; about 25 cm$^2$ to about 90 cm$^2$, about 25 cm$^2$ to about 80 cm$^2$, or about 25 cm$^2$ to about 70 cm$^2$, about 25 cm$^2$ to about 60 cm$^2$, about 25 cm$^2$ to about 50 cm$^2$, about 25 cm$^2$ to about 40 cm$^2$, or about 25 cm$^2$ to about 30 cm$^2$; preferably, the affected area(s) of the subject is/are about 0.2 cm$^2$, about 0.5 cm$^2$, about 1 cm$^2$, about 2 cm$^2$, about 3 cm$^2$, about 4 cm$^2$, about 5 cm$^2$, about 6 cm$^2$, about 7 cm$^2$, about 8 cm$^2$, about 9 cm$^2$, about 10 cm$^2$, about 11 cm$^2$, about 12 cm$^2$, about 13 cm$^2$, about 14 cm$^2$, about 15 cm$^2$, about 16 cm$^2$, about 17 cm$^2$, about 18 cm$^2$, about 19

cm$^2$, about 20 cm$^2$, about 25 cm$^2$, about 30 cm$^2$, about 35 cm$^2$, about 40 cm$^2$, about 45 cm$^2$, about 50 cm$^2$, about 55 cm$^2$, about 60 cm$^2$, about 65 cm$^2$, about 70 cm$^2$, about 75 cm$^2$, about 80 cm$^2$, about 85 cm$^2$, about 90 cm$^2$, about 95 cm$^2$, or about 100 cm$^2$.

**10.** The method according to any one of the preceding claims, **characterized in that** the affected area(s) of the subject is/are on the skin.

**11.** The method according to any one of the preceding claims, **characterized in that** the affected area(s) of the subject is/are located at one or more positions independently selected from the scalp, forehead, forearm, face, nose, ears, eyelids, lips, neck, arms, hands, trunk, legs, and feet.

**12.** The method according to any one of the preceding claims, **characterized in that** 17β-neriifolin or a pharmaceutically acceptable salt, racemate, stereoisomer, enantiomer, diastereomer, tautomer, metabolite, solvate or prodrug thereof is formulated into a pharmaceutical composition suitable for topical use.

**13.** The method according to any one of the preceding claims, **characterized in that** the content of 17β-neriifolin or a pharmaceutically acceptable salt, racemate, stereoisomer, enantiomer, diastereomer, tautomer, metabolite, solvate or prodrug thereof is about 0.025 wt% to 4 wt%; preferably about 0.05 wt% to 3 wt%; preferably about 0.2 wt% to 0.8 wt%; preferably about 0.075 wt%, about 0.1 wt%, about 0.125 wt%, about 0.15 wt%, about 0.175 wt%, about 0.2 wt%, about 0.225 wt%, about 0.25 wt%, about 0.275 wt%, about 0.3 wt%, about 0.325 wt%, about 0.35 wt%, about 0.375 wt%, about 0.4 wt%, about 0.425 wt%, about 0.45 wt%, about 0.475 wt%, about 0.5 wt%, about 0.525 wt%, about 0.55 wt%, about 0.575 wt%, about 0.6 wt%, about 0.625 wt%, about 0.65 wt%, about 0.675 wt%, about 0.7 wt%, about 0.725 wt%, about 0.75 wt%, about 0.775 wt%, about 0.8 wt%, about 0.825 wt%, about 0.85 wt%, about 0.875 wt%, about 0.9 wt%, about 0.925 wt%, about 0.95 wt%, about 0.975 wt%, about 1 wt%, about 1.25 wt%, about 1.5 wt%, about 1.75 wt%, about 2.0 wt%, about 2.25 wt%, about 2.5 wt%, or about 2.75 wt%, based on the total weight of the pharmaceutical composition.

**14.** The method according to any one of the preceding claims, **characterized in that** the amount of the pharmaceutical composition administered to an affected skin area(s) ranges/range from about 0.001 g/cm$^2$ of skin surface area to about 0.5 g/cm$^2$ of skin surface area; preferably about 0.0015 g/cm$^2$ of skin surface area to about 0.2 g/cm$^2$ of skin surface area; preferably about 0.0015 g/cm$^2$ of skin surface area to about 0.15 g/cm$^2$ of skin surface area; preferably about 0.002 g/cm$^2$ of skin surface area to about 0.125 g/cm$^2$ of skin surface area; preferably about 0.003 g/cm$^2$ of skin surface area to about 0.1 g/cm$^2$ of skin surface area; preferably about 0.004 g/cm$^2$ of skin surface area; about 0.005 g/cm$^2$ of skin surface area; about 0.006 g/cm$^2$ of skin surface area; about 0.007 g/cm$^2$ of skin surface area; about 0.008 g/cm$^2$ of skin surface area; about 0.009 g/cm$^2$ of skin surface area; about 0.01 g/cm$^2$ of skin surface area; about 0.0125 g/cm$^2$ of skin surface area; about 0.015 g/cm$^2$ of skin surface area; about 0.0175 g/cm$^2$ of skin surface area; about 0.02 g/cm$^2$ of skin surface area; about 0.03 g/cm$^2$ of skin surface area; about 0.04 g/cm$^2$ of skin surface area; about 0.05 g/cm$^2$ of skin surface area; about 0.06 g/cm$^2$ of skin surface area; about 0.07 g/cm$^2$ of skin surface area; about 0.08 g/cm$^2$ of skin surface area; or about 0.09 g/cm$^2$ of skin surface area.

**15.** The method according to any one of the preceding claims, **characterized in that** 17β-neriifolin or a pharmaceutically acceptable salt, racemate, stereoisomer, enantiomer, diastereomer, tautomer, metabolite, solvate or prodrug thereof is administered daily, at one-day intervals, at two-day intervals, at three-day intervals, at four-day intervals, at five-day intervals, at six-day intervals, or at seven-day intervals; on the day of administration, one, two, three, or four administrations are performed.

**16.** The method according to any one of the preceding claims, **characterized in that** the pharmaceutical composition is a gel.

**17.** The method according to any one of the preceding claims, **characterized in that** the pharmaceutical composition comprises a gel matrix, wherein the gel matrix is preferably selected from at least one of sodium carboxymethyl cellulose, carbomer, gelatin, and alginate; the content of the gel matrix is about 0.5 wt% to 5 wt%; preferably about 1 wt% to 4 wt%; preferably about 1 wt% to 3 wt%; preferably about 1.5 wt%, about 2 wt%, about 2.5 wt%, or about 3 wt%, based on the total weight of the pharmaceutical composition; and/or

the pharmaceutical composition comprises a humectant, wherein the humectant is selected from at least one of alcoholic humectants; preferably, said humectant is glycerol, wherein the content of the humectant is about 5 wt% to 35 wt%; preferably about 10 wt% to 30 wt%; preferably about 15 wt% to 25 wt%; more preferably about 15 wt%,

about 20 wt%, about 25 wt%, or about 30 wt%, based on the total weight of the pharmaceutical composition; and/or
the pharmaceutical composition comprises a solvent; preferably, the solvent is selected from at least one of ethanol, propylene glycol, and benzyl alcohol; more preferably, the ethanol is 95% ethanol or anhydrous ethanol; the content of the solvent is about 20 wt% to 75 wt%; preferably about 25 wt% to 70 wt%; more preferably about 30 wt%, about 35 wt%, about 40 wt%, about 45 wt%, about 50 wt%, about 55 wt%, about 60 wt%, or about 65 wt%, based on the total weight of the pharmaceutical composition; and/or
the pharmaceutical composition comprises water, preferably purified water, distilled water, or deionized water; the content of the water is about 20 wt% to 75 wt%; preferably about 25 wt% to 70 wt%; more preferably about 30 wt%, about 35 wt%, about 40 wt%, about 45 wt%, about 50 wt%, about 55 wt%, about 60 wt%, or about 65 wt%, based on the total weight of the pharmaceutical composition; and/or
the pharmaceutical composition comprises a penetration enhancer, wherein the penetration enhancer is selected from at least one of azone, borneol, Tween 80, sodium dodecyl sulfate, and poloxamer; more preferably, the penetration enhancer is azone or borneol; the content of the penetration enhancer is about 0 wt% to 5 wt%; preferably about 0.1 wt% to 4 wt%; more preferably about 0.25 wt%, about 0.5 wt%, about 0.75 wt%, about 1 wt%, about 1.25 wt%, about 1.5 wt%, about 1.75 wt%, about 2 wt%, about 2.25 wt%, about 2.5 wt%, about 2.75 wt%, about 3 wt%, about 3.25 wt%, about 3.5 wt%, or about 3.75 wt%, based on the total weight of the pharmaceutical composition.

**18.** The method according to any one of the preceding claims, **characterized in that** the pharmaceutical composition comprises a preservative; the preservative is a paraben preservative and/or butylated hydroxytoluene; preferably, the paraben preservative is methylparaben and/or ethylparaben; the content of the preservative is about 0 wt% to 4 wt%; preferably about 0.1 wt% to 3 wt%; more preferably about 0.25 wt%, about 0.5 wt%, about 0.75 wt%, about 1 wt%, about 1.25 wt%, about 1.5 wt%, about 1.75 wt%, about 2 wt%, about 2.25 wt%, about 2.5 wt%, or about 2.75 wt%, based on the total weight of the pharmaceutical composition.

**19.** The method according to any one of the preceding claims, **characterized in that** the pharmaceutical composition comprises:

about 0.2 wt% to 0.8 wt% 17β-neriifolin or a pharmaceutically acceptable salt, racemate, stereoisomer, enantiomer, diastereomer, tautomer, metabolite, solvate or
prodrug thereof; and/or
about 1.5 wt% gel matrix; and/or
about 40 wt% solvent; and/or
about 0.5 wt% penetration enhancer; and/or
about 20 wt% humectant;
the balance being water.

**20.** The method according to any one of the preceding claims, **characterized in that** compared to other treatments for skin cancer or precancerous lesions thereof, the administration of 17β-neriifolin or a pharmaceutically acceptable salt, racemate, stereoisomer, enantiomer, diastereomer, tautomer, metabolite, solvate or prodrug thereof reduces the number and/or severity of local skin reactions or additional adverse side effects in the subject.

**21.** The method according to any one of the preceding claims, **characterized in that** the local skin reaction is selected from vesicle formation, pustule formation, erosion, ulceration, redness, swelling, scaling, desquamation, induration, dryness, pus, and blistering.

1.2
1
0.8
0.6
0.4
0.2
0
Relative Cellular Activity
0
20
40
60
80
100
concentration (ng/ml)

FIG. 1

SCL-1 human skin cancer
Model control group
Drug substance group
Imiquimod cream group
Gel group I
Gel group II
Gel group III
Gel group IV
Volume（mm³）
3500
3000
2500
2000
1500
1000
500
0
0D
3D
6D
9D
12D
15D
D18
D21
Days

FIG. 2

COLO-16 human skin cancer
TUMER VOLUME（MM³）
4000
3500
3000
2500
2000
1500
1000
500
0
Model control group
Drug substance group
Imiquimod cream group
Gel group I
Gel group II
Gel group III
Gel group IV
0D
3D
6D
9D
12D
15D
18D
21D
DAYS

FIG. 3

Model control group
Drug substance group
Imiquimod cream group
Gel group I
Gel group II
Gel group III
Gel group IV

FIG. 4

**INTERNATIONAL SEARCH REPORT**

International application No.
**PCT/CN2024/124477**

**A. CLASSIFICATION OF SUBJECT MATTER**

A61K 31/7048(2006.01)i; A61P35/00(2006.01)i

According to International Patent Classification (IPC) or to both national classification and IPC

**B. FIELDS SEARCHED**

Minimum documentation searched (classification system followed by classification symbols)

IPC: A61K A61P

Documentation searched other than minimum documentation to the extent that such documents are included in the fields searched

Electronic data base consulted during the international search (name of data base and, where practicable, search terms used)

CNTXT; DWPI; ENTXT; PUBMED; ELSEVIER; NCBI; VCN; Web of Science; STNext; CJFD; CNKI; 万方, WANFANG; 读秀, DUXIU: 黄夹次甙乙, 黄夹次苷乙, 黄夹次乙苷, 皮肤癌, 黑素瘤, 黑色素瘤, 基底细胞癌, 鳞状细胞癌, 17bNF, A375, 466-07-9, neriifolin

**C. DOCUMENTS CONSIDERED TO BE RELEVANT**

| Category* | Citation of document, with indication, where appropriate, of the relevant passages | Relevant to claim No. |
|---|---|---|
| X | US 8187644 B2 (PHOENIX BIOTECHNOLOGY INC.) 29 May 2012 (2012-05-29)<br>claim 9, and embodiments 7 and 8 | 1-21 |
| X | US 2017112865 A1 (THE UNIVERSITY OF TEXAS SYSTEM) 27 April 2017 (2017-04-27)<br>claims 1-12 | 1-21 |
| X | Nurhanan M. Yunos et al. "The In Vitro Anti-Cancer Activities of 17βH-Neriifolin Isolated from Cerbera odollam and its Binding Activity on Na+, K+-ATPase"<br>*Current Pharmaceutical Biotechnology,*<br>Vol. 21, No. (1), 31 December 2020 (2020-12-31), pp. 37-44<br>abstract | 1-21 |
| A | CN 109908160 A (TIANJIN UNIVERSITY OF TRADITIONAL CHINESE MEDICINE) 21 June 2019 (2019-06-21)<br>abstract | 1-21 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **16 January 2025** | **20 January 2025** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No. **PCT/CN2024/124477**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **2-21**
because they relate to subject matter not required to be searched by this Authority, namely:

The subject matter of claims 2-21 is: a method for treating and/or preventing skin cancer or precancerous lesions thereof. The subject matter described above relates to a treatment method, and belongs to subject matter for which no search is required by the International Searching Authority as defined in PCT Rule 39.1(iv).

The above claims are reasonably expected to be amended as follows:

Claim 2. the use of a compound in the preparation of a drug for treating and/or preventing skin cancer or precancerous lesions thereof, the compound is 17β-Neriifolin or a pharmaceutically acceptable salt, racemate, stereoisomer, enantiomer, diastereoisomer, tautomer, metabolite, solvate, and prodrug thereof.

Claims 3-21. the expression "method" in the subject matter thereof is amended to "use".

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**INTERNATIONAL SEARCH REPORT**
**Information on patent family members**

International application No.
**PCT/CN2024/124477**

| Patent document cited in search report | Publication date (day/month/year) | Patent family member(s) | Publication date (day/month/year) |
|---|---|---|---|
| US 8187644 B2 | 29 May 2012 | KR 20110092360 A | 17 August 2011 |
| | | KR 101515681 B1 | 07 May 2015 |
| | | CA 2609808 A1 | 08 February 2007 |
| | | CA 2609808 C | 30 August 2016 |
| | | JP 2009502942 A | 29 January 2009 |
| | | JP 5081152 B2 | 21 November 2012 |
| | | RU 2008107585 A | 10 September 2009 |
| | | RU 2398591 C2 | 10 September 2010 |
| | | MX 2008001261 A | 25 March 2008 |
| | | KR 20080030613 A | 04 April 2008 |
| | | KR 101186089 B1 | 27 September 2012 |
| | | ES 2390843 T3 | 19 November 2012 |
| | | US 2008200401 A1 | 21 August 2008 |
| | | US 2012219620 A1 | 30 August 2012 |
| | | US 8394434 B2 | 12 March 2013 |
| | | EP 1909807 A2 | 16 April 2008 |
| | | EP 1909807 B1 | 16 May 2012 |
| | | AU 2006275891 A1 | 08 February 2007 |
| | | AU 2006275891 B2 | 30 June 2011 |
| | | WO 2007016176 A2 | 08 February 2007 |
| | | WO 2007016176 A3 | 20 November 2008 |
| | | JP 2015096546 A | 21 May 2015 |
| | | JP 6113205 B2 | 12 April 2017 |
| | | HK 1159475 A1 | 03 August 2012 |
| | | GT 200600338 A | 12 February 2007 |
| | | JP 2012197298 A | 18 October 2012 |
| | | JP 6058920 B2 | 11 January 2017 |
| | | BRPI 0614484 A2 | 29 March 2011 |
| | | AR 055358 A1 | 22 August 2007 |
| | | US 2007026092 A1 | 01 February 2007 |
| | | US 7402325 B2 | 22 July 2008 |
| US 2017112865 A1 | 27 April 2017 | WO 2015179075 A1 | 26 November 2015 |
| CN 109908160 A | 21 June 2019 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 202311331579
- CN 104736157 A